# EUROPEAN PATENT APPLICATION

(11) **EP 2 161 013 A1**
(43) Date of publication of application: **10.03.2010**
(21) Application number: 08752637.2
(22) Date of filing: 13.05.2008
(51) Int. Cl.: A61J 1/10, A61M 39/02

(54) **FEMALE CONNECTOR AND CONNECTOR**

(30) Priority: 08.06.2007 JP 2007153356
(71) Applicant: JMS Co., Ltd., Hiroshima-shi Hiroshima 730-8652 (JP)
(72) Inventor: ITO, Naotsugu, Hiroshima 730-8652 (JP); KUNISHIGE, Takahiko, Hiroshima 730-8652 (JP)
(74) Representative: Schorr, Frank Jürgen
(86) International application number: PCT/JP2008/058757
(87) International publication number: WO 2008/152871

(57) **Abstract**

A female connector (100) to be connected to a male connector (170) having a tubular section (172), out of which a liquid-like substance flows, is disclosed. The female connector has an adaptor (110) installed on the outer peripheral surface of the tubular section, and a handle (120) fitted on the adaptor. The adaptor has a tube-shaped section (112) having an opening (119a) at one end. The tube-shaped section includes an insertion section (119) into which the tubular section is inserted from the opening, and the insertion section has flexibility and elasticity. The handle has a rigidity higher than that of the insertion section of the adaptor. There is provided movement limitation means that limits a range in which the handle is capable of moving relative to the adaptor from an end of the adaptor on an opposite side of the opening to an end thereof on the opening side in a direction of a center axis (111) of the adaptor.

## Description

### Technical Field

The present invention relates to a female connector to be connected to a male connector, out of which a liquid-like substance flows. The present invention also relates to a connector composed of a male connector and a female connector.

### BackgroundArt

As a method for administering a nutrient or a drug to a patient parenterally, a transintestine nutrition therapy and an intravenous nutrition therapy are known. According to the transintestine nutrition therapy, a liquid-like substance such as a nutrient, a liquid diet, or a drug (generally called a "transintestine nutrient") is administered through a tube running from the nasal cavity to the stomach or the duodenum of a patient (generally called a "nasotracheal tube") or through a tube inserted in a gastric fistula formed in the abdomen of the patient (generally called a "PEG tube") (the operation for forming a gastric fistula is called "Percutaneous Endoscopic Gastrostomy"). Further, according to the intravenous nutrition therapy, a liquid-like substance containing a nutrient component such as glucose or a drug component (generally called an "infusion solution") is administered through an infusion solution line inserted in the vein of a patient.

The transintestine nutrition therapy through a nasotracheal tube will be described. Aliquid-like substance to be administered to a patient is stored in a medical container. A male connector, out of which the liquid-like substance flows, is provided at a lower end of the medical container. The liquid-like substance in the medical container generally is administered to a patient through a transintestine nutrient set and a nasotracheal tube. A female connector to be connected to the male connector is provided at one end of a tube constituting the transintestine nutrient set (see, for example, Patent Document 1).

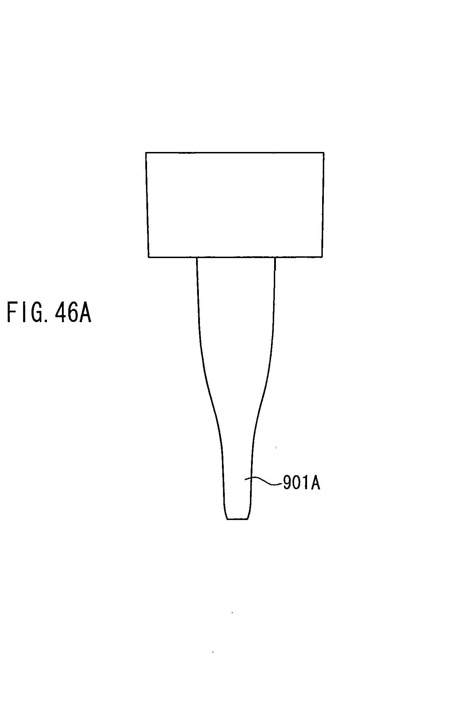

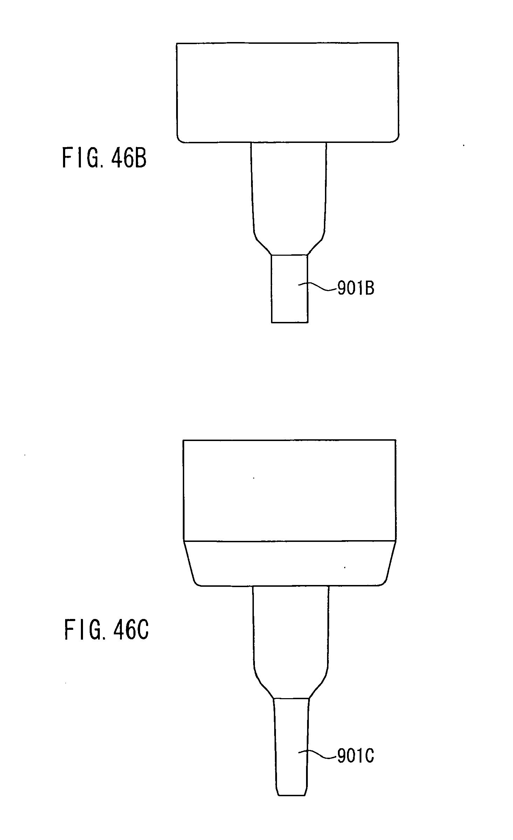

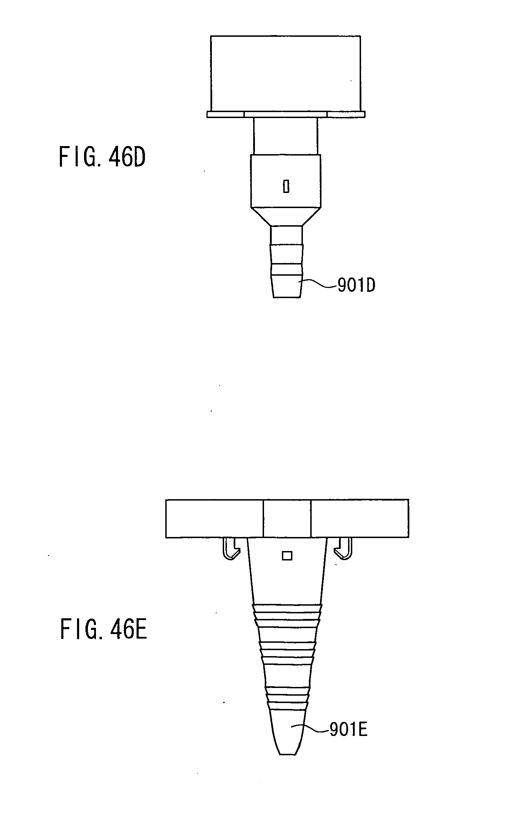

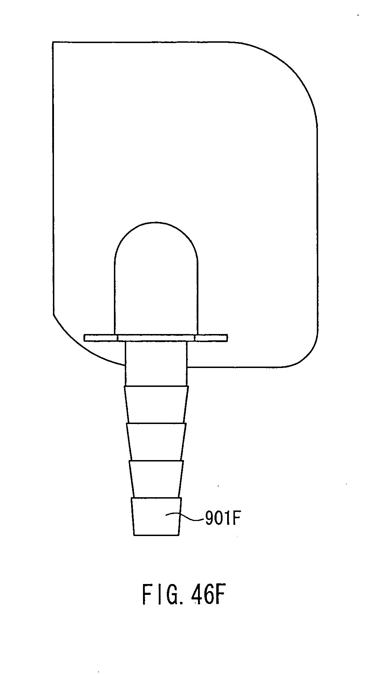

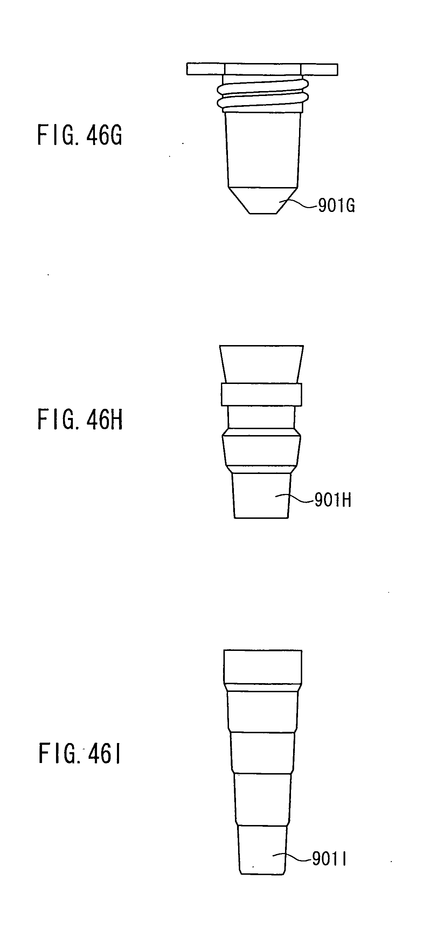

The male connector provided at the medical container generally has a tubular section, out of which the liquid-like substance flows. There are no specifications as standards regarding the shape and size of the tubular section, and the shape and size of the tubular section vary depending upon manufacturers of the medical container and the male connector. FIGS. 46A to 46I are side views showing tubular sections 901Ato 901I of conventional male connectors adopted by respective manufacturers. As shown in these figures, the specifications regarding the shape, outer diameter, length, and the like of an outer peripheral surface of the tubular section of the male connector vary depending upon manufacturers.

In a facility (for example, a medical care organization) performing transintestine nutrition, conventionally, a container to be filled with a transintestine nutrient and a transintestine nutrient set mostly are reused, and in that case, washing, disinfection, and filling of a transintestine nutrient are performed while the container and the transintestine nutrient set are connected to each other. However, recently, in view of the prevention of infection, a pre-filled nutrient, which is on sale under the condition that a disposable container is filled with a transintestine nutrient, is spreading. Along with the spread of the pre-filled nutrient, chances are growing that a container and a transintestine nutrient set are dealt with as separate bodies, and the problem of mismatching (connection defect) between a male connector of a container and a female connector of a transintestine nutrient set is becoming conspicuous, which is caused by the presence of various specifications regarding the tubular section of the male connector of the container as described above.

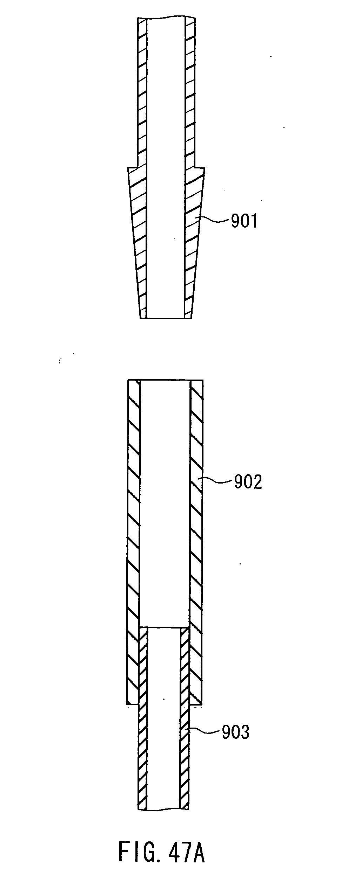

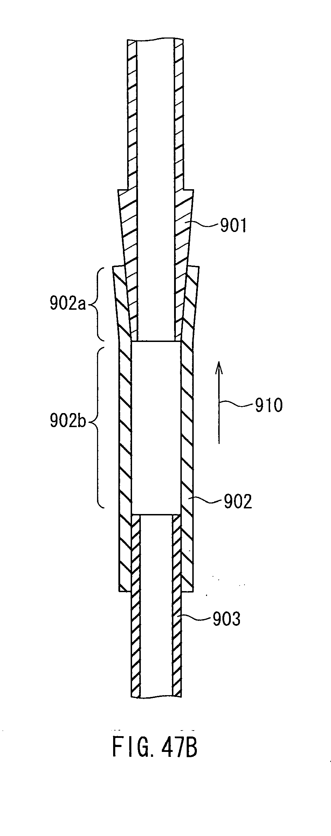

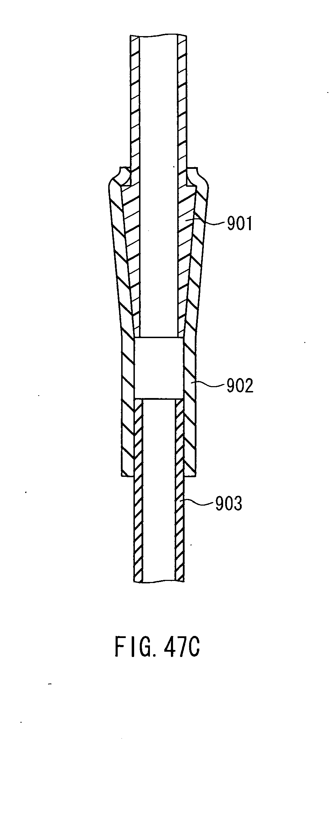

As a conventional general-purpose female connector capable of being connected to various male connectors, as shown in FIG. 47A, a cylindrical rubber tube 902 with elasticity connected to one end of a tube 903 has been used so as to be connected to a tubular section 901 of various male connectors having different shapes and sizes. As shown in FIG. 47B, the rubber tube 902 is fitted in a direction indicated by an arrow 910 so as to cover the outer peripheral surface of the tubular section 901, and as shown in FIG. 47C, the rubber tube 902 and the tubular section 901 are connected to each other. The rubber tube 902 stretches and is deformed in accordance with the tapered shape of the outer peripheral surface of the tubular section 901, and comes into tight contact with the outer peripheral surface of the tubular section 901 due to the elastic recovery force.
Patent Document 1: JP 11(1999)-28244A

### Disclosure of Invention

### Problem to be Solved by the Invention

However, the conventional general-purpose female connector made of the rubber tube 902 shown in FIG. 47Ahas a problem in that it is difficult to perform an operation of connecting the rubber tube 902 to the tubular section 901, due to the friction generated between the tubular section 901 and the rubber tube 92. In the connection operation, the outer peripheral surface of the rubber tube 902 is grasped with two fingers. In FIG. 47B, when a region 902a of the rubber tube 902 covering the tubular section 901 is grasped, the grasping force increases the friction between the tubular section 901 and the rubber tube 902, which makes it difficult to move the rubber tube 902 with respect to the tubular section 901. In FIG. 47B, when a region 902b of the rubber tube 902 not covering the tubular section 901 or the tube 903 is grasped, the rubber tube 902 is buckled and deformed easily by the force in the direction indicated by the arrow 910, which also makes it difficult to move the rubber tube 902 with respect to the tubular section 901.

When the flexibility of the rubber tube 902 is enhanced so that the rubber tube 902 elongates easily, the rubber tube 902 further becomes likely to buckle and deform, which rather makes it difficult to perform a connection operation. Further, when the inner diameter of the rubber tube 902 is increased, the friction between the tubular section 901 and the rubber tube 902 can be decreased. Therefore, the connection operation between the tubular section 901 and the rubber tube 902 becomes easy. However, in this case, there arises a new problem in that the rubber tube 902 fitted on the tubular section 901 comes off easily, or the liquid-like substance leaks from between the tubular section 901 and the rubber tube 902.

There also is a problem that it may take time and trouble to insert the tubular section 901 into the rubber tube 902 depending upon the shape and size of the outer peripheral surface of the tubular section 901 of the male connector.

The above-mentioned problems similarly occur even in the case of connecting a PEG tube or a tube constituting an infusion solution line used in the intravenous nutrition therapy.

The present invention solves the above-mentioned conventional problems, and its object is to provide a female connector that can be connected to various male connectors having different shapes and sizes and that is connected to a male connector easily, and a connector.

### Means for Solving Problem

A female connector of the present invention to be connected to a male connector having a tubular section, out of which a liquid-like substance flows, includes: an adaptor installed on an outer peripheral surface of the tubular section, and a handle fitted on the adaptor. The adaptor has a tube-shaped section having an opening at one end thereof, the tube-shaped section includes an insertion section into which the tubular section is inserted from the opening, and the insertion section has flexibility and elasticity. The handle has a rigidity higher than that of the insertion section of the adaptor. There is provided movement limitation means limiting a range in which the handle is capable of moving relative to the adaptor from an end of the adaptor on an opposite side of the opening to an end thereof on the opening side in a center axis direction of the adaptor.

A connector of the present invention includes a male connector having a tubular section, out of which a liquid-like substance flows, and a female connector to be connected to the male connector, wherein the female connector is the above-mentioned female connector of the present invention.

### Effects of the Invention

According to the present invention, since the insertion section of the adaptor has flexibility and elasticity, the female connector can be connected to various male connectors having different shapes and sizes. Further, the force in a direction approaching the male connector can be applied to the adaptor via the handle having a rigidity higher than that of the insertion section of the adaptor, and hence, the connection operation between the female connector and the male connector is easy.

### Brief Description of Drawings

[FIG. 1A] FIG. 1A is a perspective view showing a schematic configuration of a female connector according to Embodiment 1 of the present invention.
[FIG. 1B] FIG. 1B is a cross-sectional view of the female connector shown in FIG. 1A.
[FIG. 2A] FIG. 2Ais a perspective view showing a schematic configuration of an adaptor constituting the female connector according to Embodiment 1 of the present invention.
[FIG. 2B] FIG. 2B is a cross-sectional view of the adaptor shown in FIG. 2A.
[FIG. 3A] FIG. 3Ais a perspective view showing a schematic configuration of a handle constituting the female connector according to Embodiment 1 of the present invention.
[FIG. 3B] FIG. 3B is a cross-sectional view of the handle shown in FIG. 3A
[FIG. 4] FIG. 4 is an exploded view showing a schematic configuration of an example of a medical container and a male connector.
[FIG. 5A] FIG. 5Ais a cross-sectional view showing a method for connecting the female connector according to Embodiment 1 of the present invention to a male connector, illustrating a state before the connection.
[FIG. 5B] FIG. 5B is a cross-sectional view showing a method for connecting the female connector according to Embodiment 1 of the present invention to a male connector, illustrating a state after the connection.
[FIG. 6] FIG. 6 is a cross-sectional view showing a state in which the female connector according to Embodiment 1 of the present invention is connected to a male connector having a tubular section with a small outer diameter.
[FIG. 7A] FIG. 7Ais a perspective view showing a schematic configuration of another adaptor constituting the female connector according to Embodiment 1 of the present invention.
[FIG. 7B] FIG. 7B is a cross-sectional view of the adaptor shown in FIG. 7A.
[FIG. 8] FIG. 8 is a perspective view showing a schematic configuration of still another adaptor constituting the female connector according to Embodiment 1 of the present invention.
[FIG. 9A] FIG. 9Ais a perspective view showing a schematic configuration of the adaptor provided with a mechanism holding a handle in Embodiment 1 of the present invention.
[FIG. 9B] FIG. 9B is a cross-sectional view showing an operation of the mechanism holding a handle, provided on the adaptor shown in FIG. 9A.
[FIG. 10A] FIG. 10Ais a perspective view showing a schematic configuration of the adaptor provided with another mechanism holding a handle in Embodiment 1 of the present invention.
[FIG. 10B] FIG. 10B is a cross-sectional view of the adaptor taken along a line 10B-10B in FIG. 10A.
[FIG. 11] FIG. 11 is a perspective view showing a schematic configuration of the female connector provided with another mechanism holding a handle in Embodiment 1 of the present invention.
[FIG. 12] FIG. 12 is a perspective view showing a schematic configuration of the female connector provided with another mechanism holding a handle in Embodiment 1 of the present invention.
[FIG. 13A] FIG. 13Ais a perspective view showing a schematic configuration of another handle constituting the female connector according to Embodiment 1 of the present invention.
[FIG. 13B] FIG. 13B is a cross-sectional view of the handle shown in FIG. 13A.
[FIG. 14A] FIG. 14A is a perspective view showing a schematic configuration of still another handle constituting the female connector according to Embodiment 1 of the present invention.
[FIG. 14B] FIG. 14B is a perspective view showing a schematic configuration of still another handle constituting the female connector according to Embodiment 1 of the present invention.
[FIG. 15A] FIG. 15Ais a perspective view showing a schematic configuration of still another handle constituting the female connector according to Embodiment 1 of the present invention.
[FIG. 15B] FIG. 15B is a cross-sectional view of the handle shown in FIG. 15A.
[FIG. 16A] FIG. 16Ais a perspective view showing a schematic configuration of still another handle constituting the female connector according to Embodiment 1 of the present invention.
[FIG. 16B] FIG. 16B is a cross-sectional view of the handle shown in FIG. 16A.
[FIG. 17] FIG. 17 is a perspective view showing a schematic configuration of still another handle constituting the female connector according to Embodiment 1 of the present invention.
[FIG. 18A] FIG. 18Ais a perspective view showing a schematic configuration of still another handle constituting the female connector according to Embodiment 1 of the present invention.
[FIG. 18B] FIG. 18B is a plan view of the handle shown in FIG. 18A.
[FIG. 19A] FIG. 19Ais a perspective view showing a schematic configuration of still another handle constituting the female connector according to Embodiment 1 of the present invention.
[FIG. 19B] FIG. 19B is a plan view of the handle shown in FIG. 19A.
[FIG. 20A] FIG. 20Ais a perspective view showing a schematic configuration of another female connector according to Embodiment 1 of the present invention.
[FIG. 20B] FIG. 20B is a cross-sectional view of the female connector shown in FIG. 20A.
[FIG. 21A] FIG. 21Ais a perspective view showing a schematic configuration of an adaptor constituting another female connector according to Embodiment 1 of the present invention.
[FIG. 21B] FIG. 21B is a cross-sectional view of the adaptor shown in FIG. 21A.
[FIG. 22A] FIG. 22Ais a perspective view showing a schematic configuration of a handle constituting another female connector according to Embodiment 1 of the present invention.
[FIG. 22B] FIG. 22B is a cross-sectional view of the handle shown in FIG. 22A.
[FIG. 23A] FIG. 23Ais a perspective view showing a schematic configuration of still another female connector according to Embodiment 1 of the present invention.
[FIG. 23B] FIG. 23B is a cross-sectional view of the female connector shown in FIG. 23A.
[FIG. 24] FIG. 24 is a perspective view showing a schematic configuration of a male connector according to Embodiment 2 of the present invention.
[FIG. 25A] FIG. 25Ais a front view showing a schematic configuration of the male connector according to Embodiment 2 of the present invention.
[FIG. 25B] FIG. 25B is a bottom view showing a schematic configuration of the male connector according to Embodiment 2 of the present invention.
[FIG. 26] FIG. 26 is a perspective view showing a schematic configuration of a handle constituting the female connector according to Embodiment 2 of the present invention.
[FIG. 27A] FIG. 27Ais a plan view showing a schematic configuration of the handle constituting the female connector according to Embodiment 2 of the present invention.
[FIG. 27B] FIG. 27B is a front view showing a schematic configuration of the handle constituting the female connector according to Embodiment 2 of the present invention.
[FIG. 27C] FIG. 27C is a right side view showing a schematic configuration of the handle constituting the female connector according to Embodiment 2 of the present invention.
[FIG. 28A] FIG. 28Ais a cross-sectional view of the handle taken along a line 28A-28Ain FIG. 27B.
[FIG. 28B] FIG. 28B is a cross-sectional view of the handle taken along a line 28B-28B in FIG. 27C.
[FIG. 29] FIG. 29 is a cross-sectional view showing a method for connecting a female connector to a male connector in Embodiment 2 of the present invention.
[FIG. 30] FIG. 30 is a perspective view showing a state in which the female connector and the male connector according to Embodiment 2 of the present invention are connected to each other.
[FIG. 31] FIG. 31 is a perspective view showing a schematic configuration of a male connector according to Embodiment 3 of the present invention.
[FIG. 32A] FIG. 32Ais a front view showing a schematic configuration of the male connector according to Embodiment 3 of the present invention.
[FIG. 32B] FIG. 32B is a bottom view showing a schematic configuration of the male connector according to Embodiment 3 of the present invention.
[FIG. 33] FIG. 33 is a perspective view showing a schematic configuration of a handle constituting a female connector according to Embodiment 3 of the present invention.
[FIG. 34A] FIG. 34Ais a plan view showing a schematic configuration of the handle constituting the female connector according to Embodiment 3 of the present invention.
[FIG. 34B] FIG. 34B is a front view showing a schematic configuration of the handle constituting the female connector according to Embodiment 3 of the present invention.
[FIG. 35A] FIG. 35Ais a cross-sectional view of the handle taken along a line 35A-35Ain FIG. 34A.
[FIG. 35B] FIG. 35B is a cross-sectional view of the handle taken along a line 35B-35B in FIG. 34B.
[FIG. 36] FIG. 36 is a cross-sectional view showing a method for connecting a female connector to a male connector in Embodiment 3 of the present invention.
[FIG. 37] FIG. 37 is a perspective view showing a state in which the female connector and the male connector according to Embodiment 3 of the present invention are connected to each other.
[FIG. 38] FIG. 38 is a perspective view showing a schematic configuration of a male connector according to Embodiment 4 of the present invention.
[FIG. 39A] FIG. 39Ais a plan view showing a schematic configuration of the male connector according to Embodiment 4 of the present invention.
[FIG. 39B] FIG. 39B is a bottom view showing a schematic configuration of the male connector according to Embodiment 4 of the present invention.
[FIG. 40] FIG. 40 is a perspective view showing a schematic configuration of a handle constituting the female connector according to Embodiment 4 of the present invention.
[FIG. 41] FIG. 41 is a cross-sectional view taken along a center axis of the handle constituting the female connector according to Embodiment 4 of the present invention.
[FIG. 42] FIG. 42 is a cross-sectional view showing a method for connecting the female connector to the male connector in Embodiment 4 of the present invention.
[FIG. 43] FIG. 43 is a perspective view showing a state in which the female connector and the male connector according to Embodiment 4 of the present invention are connected to each other.
[FIG. 44] FIG. 44 is a view showing another exemplary schematic configuration of a medical container.
[FIG. 45A] FIG. 45Ais a schematic perspective view of a male connector (port) used in a medical container in FIG. 44.
[FIG. 45B] FIG. 45B is a front view of the male connector (port) shown in FIG. 45A.
[FIG. 45C] FIG. 45C is right side view of the male connector (port) shown in FIG. 45A.
[FIG. 46A] FIG. 46Ais a side view showing an example of a tubular section of a conventional male connector.
[FIG. 46B] FIG. 46B is a side view showing another example of the tubular section of the conventional male connector.
[FIG. 46C] FIG. 46C is a side view showing still another example of the tubular section of the conventional male connector.
[FIG. 46D] FIG. 46D is a side view showing still another example of the tubular section of the conventional male connector.
[FIG. 46E] FIG. 46E is a side view showing still another example of the tubular section of the conventional male connector.
[FIG. 46F] FIG. 46F is a side view showing still another example of the tubular section of the conventional male connector.
[FIG. 46G] FIG. 46G is a side view showing still another example of the tubular section of the conventional male connector.
[FIG. 46H] FIG. 46H is a side view showing still another example of the tubular section of the conventional male connector.
[FIG. 46I] FIG. 46I is a side view showing still another example of the tubular section of the conventional male connector.
[FIG. 47A] FIG. 47Ais a cross-sectional view showing an example of a method for connecting a conventional general-purpose female connector to a male connector, illustrating the state before the connection.
[FIG. 47B] FIG. 47B is a cross-sectional view showing an example of a method for connecting a conventional general-purpose female connector to a male connector, illustrating the state during the connection.
[FIG. 47C] FIG. 47C is a cross-sectional view showing an example of a method for connecting a conventional general-purpose female connector to a male connector, illustrating the state after the connection.

### Description of the Invention

In the above-mentioned female connector of the present invention, the movement limitation means may include a flange section provided at the end of the tube-shaped section of the adaptor on the opening side or in a vicinity thereof so as to protrude outward from the outer peripheral surface of the tube-shaped section, and a flange holding section provided on the handle so as to hold a surface of the flange section on an opposite side of the opening. Since the flange section is provided at the end of the tube-shaped section on the opening side or in the vicinity thereof, the rigidity of an edge of the opening of the tube-shaped section is enhanced. Thus, when the tubular section of the male connector is inserted into the opening of the tube-shaped section of the adaptor, the tube-shaped section is unlikely to be deformed. Further, since the tubular section can be inserted into the tube-shaped section while the flange section provided on the tube-shaped section is being held by the flange holding section of the handle, the tube-shaped section can be prevented from being buckled and deformed. Thus, according to the preferred embodiment, the insertion operation of the tubular section into the adaptor is facilitated further.

In the above-mentioned female connector of the present invention, the movement limitation means further may limit a range in which the handle is capable of moving relative to the adaptor from the end of the adaptor on the opening side to the end thereof on the opposite side of the opening in the center axis direction of the adaptor. This allows the handle to be held by the adaptor at all times, so that the handle can be prevented from moving to a position far away from the adaptor. Thus, the operability for connecting the female connector to the male connector is enhanced, and further, the handle can be prevented from being damaged accidentally in the production step of a transintestine nutrient set, a PEG tube, or the like.

In this case, it is preferred that the movement limitation means includes an annular groove in a circumferential direction, provided on a surface of one of the tube-shaped section and the handle opposed to the other, and a fitting section provided on the other of the tube-shaped section and the handle so as to be fitted in the groove. This can provide the movement limitation means easily at a low cost.

In this case, it is preferred that, when a width of the groove is defined as W1 and a width of the fitting section is defined as W2 in the center axis direction of the adaptor, 1.0 ≤ W1/W2 ≤ 1.5 is satisfied. When W1/W2 is smaller than the lower limit, it is difficult to fit the fitting section in the groove. When W1/W2 is larger the upper limit, the movable range of the handle relative to the adaptor is enlarged, so that the center axis of the handle is likely to tilt with respect to the center axis of the adaptor, which makes it necessary to perform a complicated operation such as the correction of the direction of the tilting handle, and which may cause the fitting section to come off the groove.

In the above-mentioned female connector of the present invention, the movement limitation means may include a bonding section that bonds the adaptor to the handle. This also can realize the movement limitation means limiting a range, in which the handle is capable of moving relative to the adaptor in a direction of the center axis of the adaptor, easily at a low cost.

In the above-mentioned female connector of the present invention, it is preferred that an inner peripheral surface of the insertion section of the adaptor has a tapered surface whose inner diameter increases toward the opening. According to the above configuration, even when the shape, size, and the like of the tubular section of the male connector vary, at least a part of the outer peripheral surface of the tubular section of the male connector and at least a part of the inner peripheral surface of the insertion section of the adaptor can be brought into tight contact with each other. Therefore, the female connector can be connected to various male connectors precisely without the leakage of a liquid-like substance.

In the above-mentioned female connector of the present invention, it is preferred that an inner peripheral surface of the insertion section of the adaptor is provided with an annular protrusion in a circumferential direction. According to the above configuration, the annular protrusion of the insertion section can be deformed appropriately and brought into tight contact with the outer peripheral surface of the tubular section, depending upon the shape, size, and the like of the tubular section of the male connector. Thus, the female connector can be connected to various male connectors precisely without the leakage of a liquid-like substance.

In the above-mentioned female connector of the present invention, it is preferred that an inner peripheral surface of the insertion section of the adaptor is provided with an annular difference in level in a circumferential direction, and the difference in level changes an inner diameter of the insertion section in a stepped shape so that the inner diameter of the insertion section on the opening side with respect to the difference in level becomes larger than the inner diameter of the insertion section on an opposite side of the opening with respect to the difference in level. According to the above configuration, in the case where the tubular section of the male connector is thick, the tubular section comes into tight contact with a portion of the insertion section of the adaptor on the opening side with respect to the difference in level, and in the case where the tubular section of the male connector is thin, the tubular section comes into tight contact with a portion of the insertion section of the adaptor on an opposite side of the opening with respect to the difference in level. Thus, the female connector can be connected to the tubular section having various thicknesses precisely without the leakage of a liquid-like substance.

In the above-mentioned female connector of the present invention, it is preferred that the adaptor is provided with a holding mechanism holding the handle. This can prevent the handle from moving to a position far away from the adaptor. Thus, the operability for connecting the female connector to the male connector is enhanced, and further, the handle can be prevented from being damaged accidentally in the production step of a transintestine nutrient set, a PEG tube, or the like.

In the above, the holding mechanism may be a locking protrusion that protrudes outward from the outer peripheral surface of the tube-shaped section of the adaptor so as to abut on an end of the handle on an opposite side of the male connector. Alternatively, the holding mechanism may be a contact protrusion that protrudes outward from the outer peripheral surface of the tube-shaped section of the adaptor so as to abut on an inner peripheral surface of the handle. In any case, the holding mechanism can be realized with a simple configuration. Further, since the holding mechanism can be molded integrally with the adaptor, so that an adaptor with a holding mechanism can be created inexpensively and efficiently.

In the above-mentioned female connector of the present invention, a tube connected to an end of the adaptor on an opposite side of the opening may be provided with a holding mechanism holding the handle. Alternatively, the handle may be provided with a holding mechanism so as to be held by a tube connected to an end of the adaptor on an opposite side of the opening or the adaptor. In any case, the handle can be prevented from moving to a position far away from the adaptor. Thus, the operability for connecting the female connector to the male connector is enhanced, and further, the handle can be prevented from being damaged accidentally in the production step of a transintestine nutrient set, a PEG tube, or the like.

In the above-mentioned female connector of the present invention, it is preferred that the handle has an annular shape surrounding the adaptor without any rift. This enhances the rigidity of the handle. Thus, the force in a diameter direction applied to the handle when two fingers grasp the handle for connecting the female connector to the male connector is unlikely to be transmitted. Therefore, the friction between the adaptor and the tubular section of the male connector will not increase. Thus, the connection operation of the female connector with respect to the male connector becomes easy. Further, by bringing at least a part of the inner peripheral surface of the handle into tight contact with the outer peripheral surface of the insertion section while the tubular section of the male connector is being inserted in the insertion section of the adaptor, the insertion section can be pinched strongly (squeezed) by the tubular section and the handle. Consequently, the connection strength between the tubular section and the adaptor is enhanced, and the adaptor can be prevented from coming off accidentally to enhance stability.

In this case, an inner peripheral surface of the handle may have a tapered surface whose inner diameter decreases toward the male connector. Alternatively, an inner peripheral surface of the handle may have a small-diameter section whose inner diameter is smaller than that of the other sections. The outer peripheral surface of the tubular section of the male connector has a taped surface that becomes thinner toward the tip end side thereof in most cases. The inner peripheral surface of the handle has a tapered surface whose taper direction (a direction in which the diameter decreases gradually) is opposite to that of the outer peripheral surface of the tubular section and has a small-diameter section, whereby a part of the inner peripheral surface of the handle locally can be brought into tight contact with the outer peripheral surface of the insertion section while the tubular section of the male connector is being inserted in the insertion section of the adaptor. As a result, the insertion section is pinched further strongly by the tubular section and the handle, so that the connection strength between the tubular section and the adaptor is enhanced further.

In the above-mentioned female connector of the present invention, the handle may be provided with a slit connecting an upper end to a lower end thereof so as to be discontinuous in a circumferential direction of the adaptor. According to the above configuration, the female connector can be connected even to a male connector having a thicker tubular section. Further, if the width of the slit is set to be large, a female connector, with respect to which the handle can be attached/detached, is obtained Thus, in the case where the handle is not required or is not matched with a male connector, the handle can be removed, and thereafter, be attached again, if required.

It is preferred that a hinge is formed at a position to which the slit of the handle is opposed, and two halves constituting the handle are connected rotatably via the hinge. According to the above configuration, the female connector can be connected to even a male connector having a thicker tubular section. Further, a female connector, with respect to which the handle can be attached/detached, is obtained.

In this case, the handle may include diameter expansion limitation means that limits a diameter expansion of the handle. According to the above configuration, the insertion section can be pinched strongly to be squeezed by the tubular section and the handle.

In the above-mentioned female connector of the present invention, it is preferred that the handle includes an engagement shape capable of being engaged with the male connector. According to the above configuration, the adaptor connected to the tubular section of the male connector can be prevented from coming off the tubular section accidentally.

In this case, it is preferred that the handle includes a brim section that protrudes on the male connector side with respect to the movement limitation means, and the engagement shape is provided at the brim section. The engagement shape is provided at the brim section that protrudes on the male connector side with respect to the movement limitation means, whereby the male connector and the female connector can be engaged with each other easily.

In this case, it is preferred that an inner peripheral surface of the brim section opposed to a center axis of the handle includes an engagement wall that extends in a circumferential direction, a concave portion provided on an opposite side of the engagement wall with respect to the male connector, and a convex portion provided adjacent to the concave portion on either one side in the circumferential direction with respect to the concave portion, and the engagement shape includes the engagement wall.

In this case, it is preferred that the female connector and the following male connector are combined to constitute a connector. Specifically, the male connector has a platform in which the tubular section protrudes from a center thereof, and an outer peripheral surface of the platform is provided with an engagement tab protruding in a radial direction with respect to a center axis. Then, the engagement tab is stored in the concave portion of the handle, and the engagement tab is engaged with the engagement wall of the handle.

According to the above configuration, the male connector and the handle can be engaged with each other and the engagement can be cancelled merely by rotating the handle slightly around the center axis with respect to the male connector. Then, the adaptor connected to the tubular section of the male connector can be prevented from coming off the tubular section accidentally by engaging the male connector and the handle with each other. Further, since the handle is provided with a convex portion adjacent to a concave portion, if the engagement tab of the male connector is inserted in the concave portion and further is moved to abut on the convex portion, the engagement tab and the engagement wall can be engaged with each other precisely.

In the above configuration, it is preferred that the engagement tab of the male connector abuts on the convex portion of the handle to regulate a rotation of the handle with respect to the male connector. According to the above configuration, the male connector and the handle can be locked together precisely by a simple operation of rotating the handle with respect to the male connector until the rotation is restricted.

Further, it is preferred that the engagement tab is provided along a surface of the platform, on which the tubular section is provided. This can reduce the size of the male connector and the handle in the center axis direction.

In the above-mentioned female connector of the present invention, it is preferred that the handle includes a frame having the movement limitation means, a pair of clips protruding on the male connector side with respect to the frame, and a pair of operation sections protruding on an opposite side of the male connector with respect to the frame, wherein a surface on a side opposed to each of the pair of clips is provided with an engagement tab, and when the frame is deformed elastically so as to narrow an interval between tip ends of the pair of operation sections, an interval between tip ends of the pair of clips is widened.

In this case, it is preferred that the female connector and the following male connector are combined to constitute a connector. Specifically, the male connector has a platform in which the tubular section protrudes from a center thereof, and an engagement protrusion protruding in a radial direction with respect to a center axis is provided continuously over the outer peripheral surface of the platform. Then, the engagement tab of the handle is engaged with the engagement protrusion.

According to the above configuration, the male connector and the handle can be engaged with each other and the engagement can be cancelled merely by moving the handle with respect to the male connector in the center axis direction. Then, the adaptor connected to the tubular section of the male connector can be prevented from coming off the tubular section accidentally by engaging the male connector and the handle with each other.

In the above configuration, it is preferred that the engagement protrusion is provided along a surface of the platform, on which the tubular section is provided. This can reduce the size of the male connector and the handle in the center axis direction.

In the female connector of the present invention, it is preferred that the handle includes a female thread on the male connector side with respect to the movement limitation means.

In this case, it is preferred that the female connector and the following male connector are combined to constitute a connector. Specifically, the male connector has a platform in which the tubular section protrudes from a center thereof, and a male thread is formed on an outer peripheral surface of the platform. Then, the female thread of the handle is screwed with the male thread.

According to the above configuration, the male connector and the handle can be engaged with each other and the engagement can be cancelled merely by rotating the handle around the center axis with respect to the male connector. Then, the adaptor connected to the tubular section of the male connector can be prevented from coming off the tubular section accidentally by engaging the male connector and the handle with each other.

It is preferred that the liquid-like substance is a transintestine nutrient in a transintestine nutrition therapy In this case, for example, in a transintestine nutrition therapy through a nasotracheal tube, the female connector can be provided at one end of a transintestine nutrient set, the other end of which is connected to a nasotracheal tube inserted in the nose of a patient, and the male connector can be provided at a container storing the transintestine nutrient. Alternatively, in a transintestine nutrition therapy through a PEG tube, the female connector can be provided at one end of the PEG tube, the other end of which is inserted in the gastric fistula of a patient, and the male connector can be provided at a terminating end of a transintestine nutrient set connected to a container storing the transintestine nutrient.

A connector of the present invention includes a male connector having a tubular section, out of which a liquid-like substance flows, and a female connector to be connected to the male connector, and the female connector is the above-mentioned female connector of the present invention.

In the above-mentioned connector of the present invention, it is preferred that, when the tubular section of the male connector is inserted into the insertion section of the adaptor of the female connector, the insertion section is squeezed by the tubular section and the handle. According to the above configuration, the insertion section is pinched strongly by the tubular section and the handle, whereby the connection strength between the tubular section and the adaptor is enhanced, so that the adaptor can be prevented from coming off the tubular section accidentally to enhance the safety.

In the above-mentioned connector of the present invention, it is preferred that the male connector and the handle of the female connector are provided with engagement shapes that are engaged with each other. According to the above configuration, the adaptor connected to the tubular section of the male connector can be prevented from coming off the tubular section accidentally.

It is preferred that the connector is provided on a line connecting a container storing a transintestine nutrient in a transintestine nutrition therapy to a patient.

Hereinafter, the present invention will be described in detail by way of preferred embodiments in a transintestine nutrition therapy through a nasotracheal tube. It should be noted that the following embodiments merely are examples obtained by embodying the present invention, and the present invention is not limited thereto.

### (Embodiment 1)

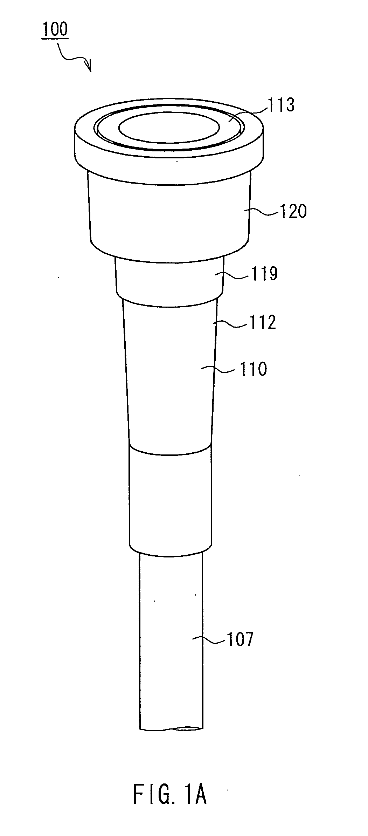

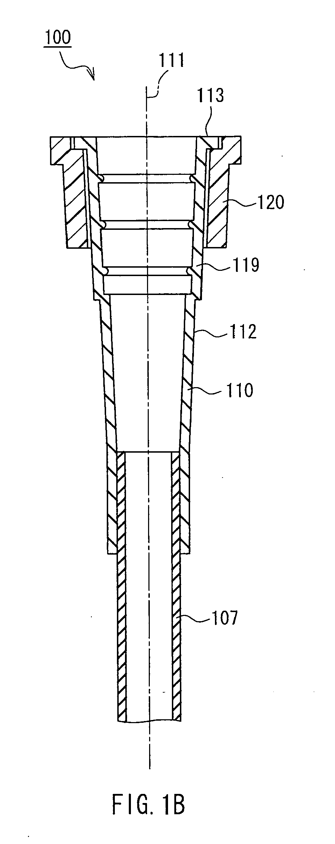

FIG. 1A is a perspective view showing a schematic configuration of a female connector 100 according to Embodiment 1 of the present invention, and FIG. 1B is a cross-sectional view thereof. The female connector 100 of Embodiment 1 includes an adaptor 110, and a handle 120 fitted on the adaptor 110. In FIG. 1B, alternate long and short dashed lines 111 indicate a center axis of the adaptor 110, which is matched with a center axis of the female connector 100 and a center axis of the handle 120. The direction of the center axis 111 is defined as an up-and-down direction, and an upper side on the drawing surfaces of FIGS. 1A and 1B (a side connected to a male connector described later) will be referred to as an "upper side" and a lower side on the drawing surfaces of the figures will be referred to as a "lower side".

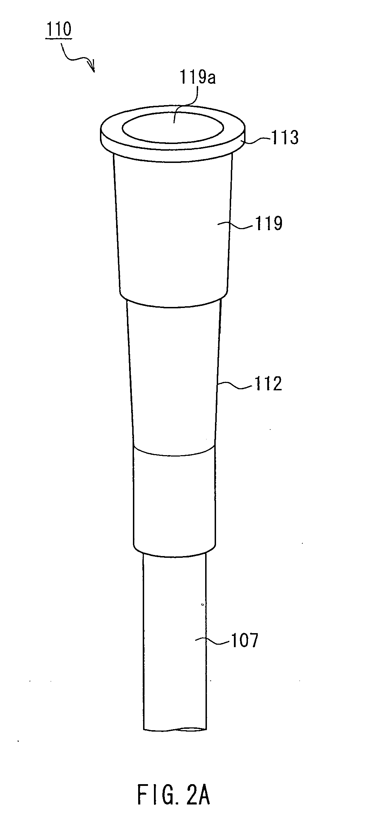

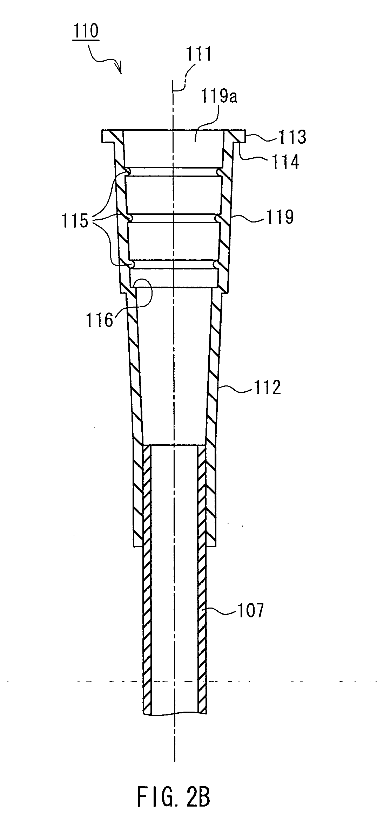

FIG. 2Ais a perspective view showing a schematic configuration of the adaptor 110 constituting the female connector 100 according to Embodiment 1, and FIG. 2B is a cross-sectional view thereof. The adaptor 110 includes a tube-shaped section 112 in a hollow cylindrical shape, and an annular flange section 113 that is formed at an upper end of the tube-shaped section 112 and protrudes outward (in a direction orthogonal to the center axis 111) from an outer peripheral surface of the tube-shaped section 112. A tube 107 having flexibility is fused to a lower end of the tube-shaped section 112. At an upper end of the tube-shaped section 112, an opening 119a in which a tubular section of the male connector is inserted is formed. It is planned that the tubular section of the male connector is inserted in the direction of the center axis 111. A region of the tube-shaped section 112 including an end on the opening 119a side will be referred to as an insertion section 119. The adaptor 110 is made of a material having flexibility and elasticity, and examples of the material include but are not particularly limited to thermoplastic resin such as natural rubber, synthetic rubber (e.g., PBD (polybutadiene)), PVC (polyvinyl chloride resin), ABS (acryl-butadiene-styrene copolymer), polyamide resin, and EVA (ethylene-vinyl acetate copolymer), polyurethane resin, and the like. Due to the use of such a material, portions (including the insertion section 119 and the flange section 113) other than the portion to which the tube 107 is fused have flexibility and elasticity.

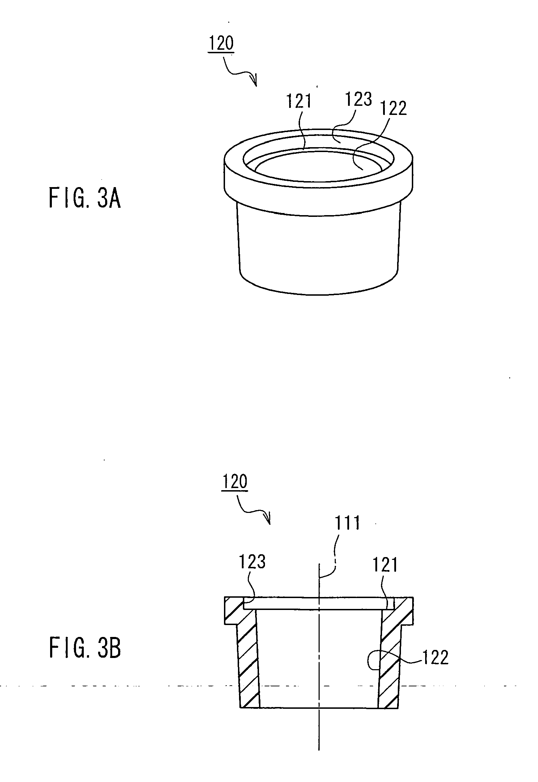

FIG. 3Ais a perspective view showing a schematic configuration of the handle 120 constituting the female connector according to Embodiment 1 of the present invention, and FIG. 3B is across-sectional view thereof. The handle 120 has a substantially tubular shape as a whole, and has a large-diameter section 123 with an inner diameter larger than an outer diameter of the flange section 113 at the upper end thereof and a small-diameter section 122 with an inner diameter larger than the outer diameter of the tube-shaped section 112 of the adaptor 110 and smaller than the outer diameter of the flange section 113 on the lower side of the large-diameter section 123. Then, a flange holding section 121 is formed between the small-diameter section 122 and the large-diameter section 123. The flange holding section 121 has a surface orthogonal to the center axis 111. The handle 120 is made of a material having a rigidity and a hardness higher than those of the adaptor 120, and examples of the material include but are not particularly limited to polypropylene resin, polycarbonate, polyacetal, PVC, and PBD.

As shown in FIGS. 1A and 1B, the adaptor 110 is inserted in a through-hole at the center of the handle 120, whereby the female connector 100 of Embodiment 1 is configured. At this time, the handle 120 surrounds the periphery of the insertion section 119 of the adaptor 110. Further, the flange section 113 of the adaptor 110 is stored in the large-diameter section 123 of the handle 120, and a lower surface 114 of the flange 113 (a surface of the flange section 113 on a side opposite to the surface opposed to the male connector) abuts on the flange holding section 121 of the handle 120. As is apparent from the above, the flange section 113 and the flange holding section 121 function as movement limitation means that sets a terminating end on an upper side (upper limit position) in a range in which the handle 120 can move relative to the adaptor 110 in the direction of the center axis 111.

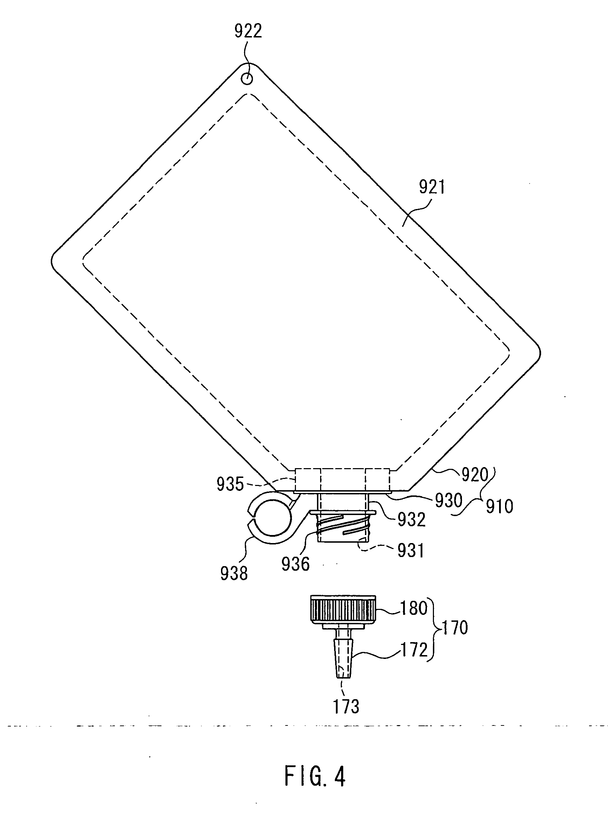

FIG. 4 is an exploded view showing a schematic configuration of an example of a medical container 910 and a male connector 170.

The medical container 910 includes a pouch 920, and a port 930 for injecting a liquid-like substance into the pouch 920 or taking out the liquid-like substance stored in the pouch 920.

The pouch 920 is a bag-shaped material obtained by laminating two flexible sheets with the same size and joining them to each other in a seal region 921 at a peripheral edge by a method such as heat seal. As the sheets, composite sheets of two or more layers can be used, which are made of materials different from each other selected from plastic materials such as polyethylene terephthalate, nylon, polypropylene, and polyethylene. Further, a thin film of aluminum oxide, silica, or the like may be formed on the sheet as a barrier layer. It is preferred that at least one of the two sheets is transparent or semi-transparent so that the amount of the liquid-like substance in the pouch 920 and the like can be checked. In the vicinity of a position, in the seal region 921 of the pouch 920, farthest from a position where the port 930 is attached, an opening 922 used for hanging the medical container 910 is formed.

The port 930 includes a cylindrical section 932 in which a through-hole 931 for passing the liquid-like substance is formed, a joint section 935 provided on an outer peripheral surface on one end side of the cylindrical section 932, and a male thread section 936 formed on the outer peripheral surface on the other end side of the cylindrical section 932. The port 930 further includes a locking section 938 in a substantially C-shape used for locking a medical tube having flexibility or hanging the medical container 910. The port 930 is made of a material relatively harder than the pouch 920, such as polyethylene, polypropylene, polyvinyl chloride, polyethyleneterephthalate, an ethylene-vinyl acetate copolymer, a thermoplastic elastomer, or polyacetal, and can be produced integrally, for example, by injection molding. The joint section 935 has a quadratic prism shape with a bottom surface having a substantially diamond shape, and is joined to be integrated with the pouch 920 by heat seal with the joint section 935 interposed between the peripheral edges of the two sheets constituting the pouch 920.

The male connector 170 has a cap section 180 and a tubular section 172. A female thread (not shown) to be screwed with the male thread section 936 of the port 930 is formed on an inner peripheral surface of the cap section 180. The tubular section 172 protrudes downward from the center on the lower surface of the cap section 180. The outer peripheral surface of the tubular section 172 of the present example has a tapered surface (truncated cone surface) whose outer diameter increases toward the cap 180. The tubular section 172 has a through-hole 172, out of which the liquid-like substance in the pouch 920 flows. There is no particular limit to the material for the male connector 170, and a known material used conventionally can be used. For example, polyolefin resin such as polypropylene or polyethylene, polycarbonate resin, or the like can be used.

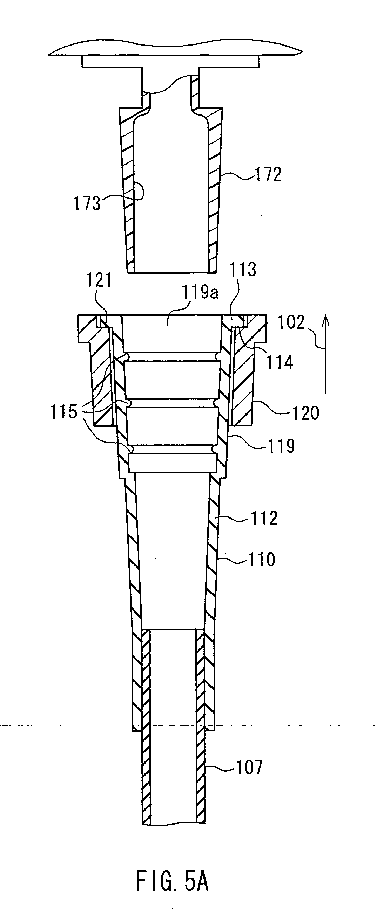

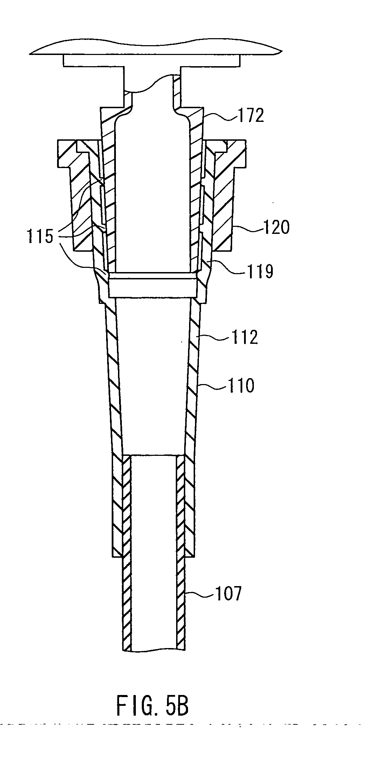

A method for connecting the female connector 100 to the male connector 170 in Embodiment 1 will be described. FIG. 5Ais a cross-sectional view showing a state before the connection, and FIG. 5B is a cross-sectional view showing a state after the connection. As shown in FIG. 5A, the adaptor 110 is inserted into a through-hole at the center of the handle 120, and the lower surface 114 of the flange section 113 of the adaptor 110 is held by the flange holding section 121 of the handle 120. In this state, the outer peripheral surface of the handle 120 is grasped with two fingers, and the adaptor 110 is installed on the tubular section 172 in a direction indicated by an arrow 102 so that the adaptor 110 covers the outer peripheral surface of the tubular section 172 of the male connector 170.

The flange section 113 is formed at the upper end of the tube-shaped section 112 of the adaptor 110, and hence, the rigidity of the peripheral edge of the opening 119a of the tube-shaped section 112 is enhanced relatively. Thus, even if the tip end of the tubular section 172 collides against the edge of the opening 119a when the tubular section 172 is inserted into the opening 119a, the tube-shaped section 112 is unlikely to be deformed. This enables the tubular section 172 to be inserted into the opening 119a easily.

Further, the insertion section 119 of the adaptor 110 has flexibility and elasticity. Therefore, the insertion section 119 is deformed, e.g., its diameter is enlarged in accordance with the shape of the outer peripheral surface of the tubular section 172 of the male connector 170, and the insertion section 119 comes into tight contact with the outer peripheral surface of the tubular section 172 due to the elastic recovery force.

On the other hand, the handle 120 hardly is deformed due to its high rigidity even when pinched with two fingers. Therefore, the grasping force applied to the handle 120 in a diameter direction by the two fingers hardly is transmitted to the insertion section 119 of the adaptor 110. Thus, unlike the conventional connection operation of the rubber tube 902, the grasping force of the two fingers will not increase the friction between the insertion section 119 of the adaptor 110 and the tubular section 172.

Further, the force applied to the handle 120 in the direction indicated by the arrow 102 by the two fingers is transmitted to the flange section 113 of the adaptor 110 via the flange holding section 121 of the handle 120. The flange section 113 is formed at a front end of the adaptor 120 in the direction indicated by the arrow 102, and hence, the adaptor 110 will not be buckled and deformed by the force.

As described above, in Embodiment 1, it is easy to move the adaptor 110 in the direction indicated by the arrow 102 with respect to the tubular section 172 of the male connector 170. Thus, as shown in FIG. 5B, the male connector 170 and the female connector 100 can be connected to each other while the insertion section 119 of the adaptor 110 is in tight contact with the outer peripheral surface of the tubular section 172 due to the elastic recovery force thereof. Even if the shape, size (for example, a taper angle, an outer diameter, etc.) and the like of the outer peripheral surface of the tubular section 172 of the male connector 170 vary depending upon manufacturers and specifications, since the insertion section 119 of the adaptor 110 is deformed elastically in accordance with the outer peripheral surface of the tubular section 172 of the male connector 170, the male connector 170 and the female connector 100 can be connected to each other precisely without the leakage of the liquid-like substance.

It is preferred that the inner peripheral surface of the insertion section 119 of the adaptor 110 is a tapered surface whose inner diameter increases toward the upper end, as shown in FIG. 2B. According to this configuration, even if the shape, size (for example, a taper angle, an outer diameter, etc.) and the like of the outer peripheral surface of the tubular section 172 of the male connector 170 vary depending upon manufacturers and specifications, at least a part of the outer peripheral surface of the tubular section 172 can be brought into tight contact with at least a part of the inner peripheral surface of the insertion section 119, so that the male connector 170 and the female connector 100 can be connected to each other precisely without the leakage of the liquid-like substance.

It is preferred that continuous annular protrusions 115 in a circumferential direction (i.e., along a circle formed by a plane orthogonal to the center axis 111 and the inner peripheral surface of the insertion section 119) are formed on the inner peripheral surface of the insertion section 119 of the adaptor 110, as shown in FIG. 2B. According to this configuration, as shown in FIG. 5B, when the adaptor 110 is connected to the tubular section 172 of the male connector 170, the protrusions 115 come into tight contact with the outer peripheral surface of the tubular section 172 easily to be deformed elastically. The degree of the deformation varies depending upon the shape, size (for example, a taper angle, an outer diameter, etc.), and the like of the tubular section 172 of the male connector 170. Thus, the protrusions 115 on the inner peripheral surface of the insertion section 119 can be deformed appropriately to be brought into tight contact with the outer peripheral surface of the tubular section 172, depending upon the shape, size and the like of the outer peripheral surface of the tubular section 172 of the male connector 170. It is preferred that the annular protrusions 115 are provided at a plurality of positions in the direction of the center axis 111, as shown in FIG. 2B. According to this configuration, even if the shape, size, and the like of the outer peripheral surface of the tubular section 172 of the male connector 170 vary depending upon manufacturers and specifications, at least one of a plurality of protrusions 115 can be brought into tight contact with the outer peripheral surface of the tubular section 172 to be deformed, depending upon the shape, size, and the like of the tubular section 172. Needless to say, one annular protrusion 115 may be used.

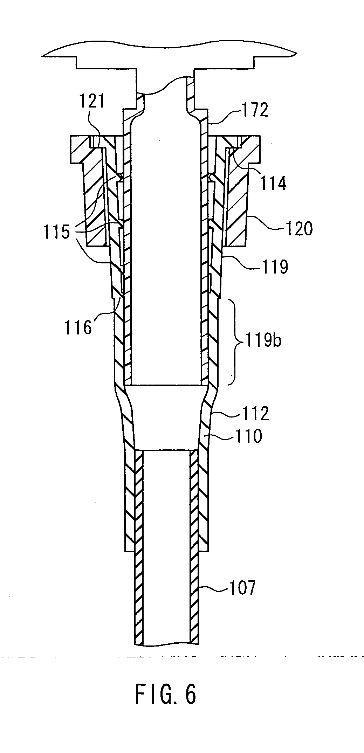

As shown in FIG. 2B, it is preferred that a difference in level 116, which changes the inner diameter of the inner peripheral surface of the insertion section 119 of the adaptor 110 in a step shape so that the inner diameter becomes smaller at the lower end side compared with that at the upper end side, is formed on the inner peripheral surface in an annular shape continuously in the circumferential direction. According to this configuration, even if the tubular section 172 is thin, the adaptor 110 can be connected thereto. That is, as shown in FIG. 6, the outer peripheral surface of the thin tubular section 172 comes into tight contact with a portion 119b with a smaller inner diameter on the lower end side of the difference in level 116 of the inner peripheral surface of the insertion section 119 of the adaptor 110. This enlarges the range of the shape, size, and the like of the outer peripheral surface of the tubular section 172 of the male connector 170 that can be connected to the female connector. It is preferred that a portion of the inner peripheral surface of the insertion section 119 on the lower end side of the difference in level 116 has a tapered surface whose inner diameter increases toward the upper end, in the same way as in a portion on the upper end side of the difference in level 116. Further, a continuous annular protrusion in the circumferential direction may be formed. A plurality of differences in level 116, which change the inner diameter in a step shape, may be provided in the direction of the center axis 111.

The above embodiment is an example, and the present invention is not limited thereto.

For example, the inner peripheral surface of the insertion section 119 of the adaptor 110 may be a cylindrical surface with an inner diameter being constant in the direction of the center axis 111, instead of the tapered surface. Further, the inner peripheral surface of the insertion section 119 of the adaptor 110 may not be provided with the protrusions 115. Further, the difference in level 116 may not be formed on the inner peripheral surface of the insertion section 119 of the adaptor 110. In any case, since the insertion section 119 of the adaptor 110 is deformed elastically depending upon the shape, size, and the like of the tubular section 172 of the male connector 170, the male connector 170 and the female connector 100 can be connected to each other precisely without the leakage of the liquid-like substance.

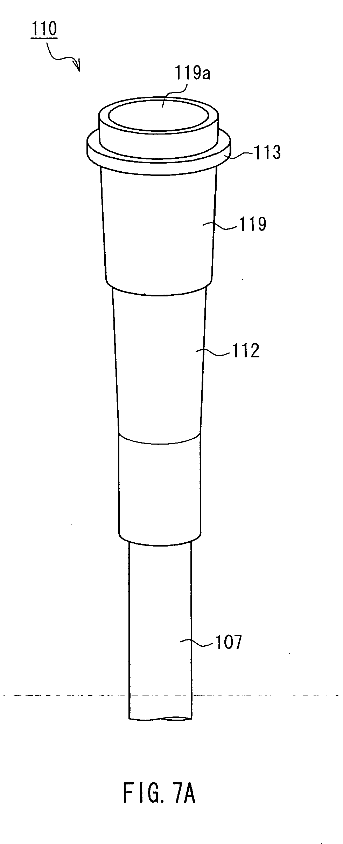

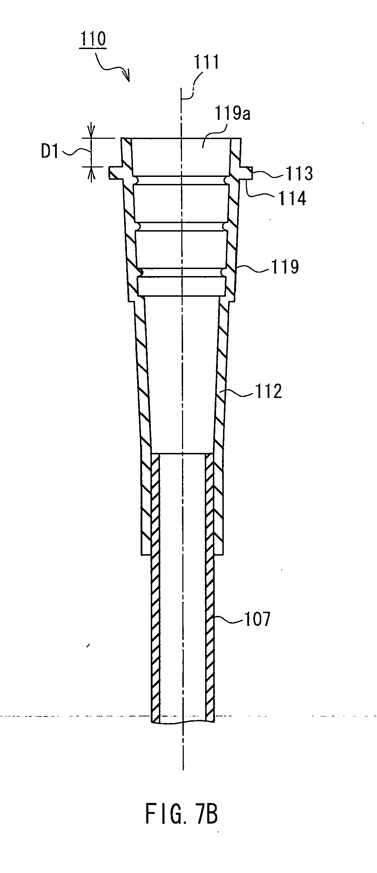

Further, the flange section 113 does not need to be provided at the upper end of the adaptor 110 in the direction of the center axis 111 as shown in FIGS. 2A and 2B. The flange section 113 may be provided at a position on a lower end side slightly away from the upper end of the adaptor 110 as shown in FIGS. 7A and 7B. As long as the flange section 113 is formed in the vicinity of the upper end of the adaptor 110, the adaptor 110 can be installed on the tubular section 172 of the male connector 170 without being buckled and deformed. If a distance D1 from the upper end of the adaptor 110 to the flange 113 is too large, a portion from the upper end of the adaptor 110 to the flange 113 is likely to be buckled and deformed when the adaptor 110 is installed on the tubular section 172 of the male connector 170. The distance D1 can be set appropriately considering the mechanical characteristics and the like of the adaptor 110, and generally is set to be 10.0 mm or less, preferably 5.0 mm or less.

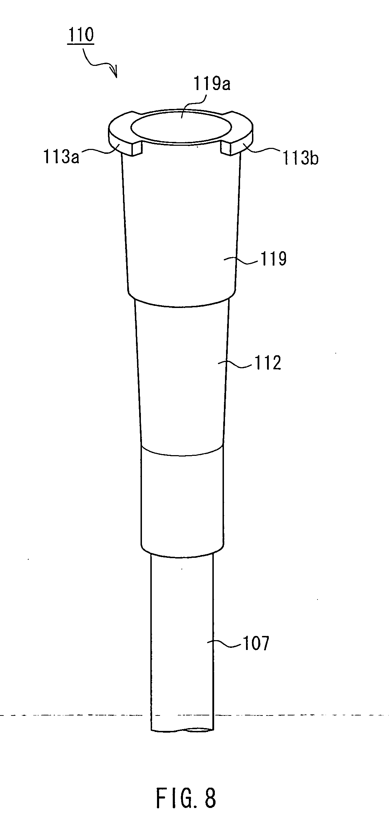

Further, it is not necessary that the flange section 113 is formed in an annular shape continuously in the circumferential direction of the adaptor 110, and the flange section 113 may be formed, for example, as two flange sections 113a, 113b shown in FIG. 8 independently in the circumferential direction. In the case where the flange section 113 is formed so as to be divided in the circumferential direction, the number thereof is not limited to two as shown in FIG. 8. The flange section 113 may be divided into three, four, or five or more.

In the female connector 100 shown in the above embodiment, when the hand is taken off the handle 120 after the adaptor 110 of the female connector 100 is connected to the tubular section 172 of the male connector 170 as shown in FIG. 5B, there is a possibility that the handle 120 may drop downward. Since the tube 107 passes through a through-hole at the center of the handle 120, the handle 120 will not drop from the tube 107. However, when the handle 120 moves to a position far away from the adaptor 110, an operation becomes complicated, for example, in the case where the adaptor 110 is attached/detached repeatedly with respect to the tubular section 172 of the male connector 170. Further, in the course of production of a transintestine nutrient set, when the handle 120 moves to a position away from the adaptor 110, an external force is applied to the handle 120 accidentally when handling of the transintestine nutrient set or the like is performed, with the result that the handle 120 may be broken. Thus, it is preferred that a mechanism holding the handle 120 at a desired position is provided at the female connector.

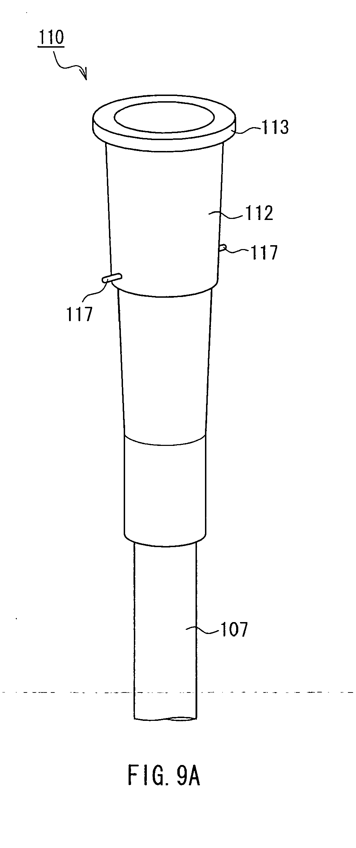

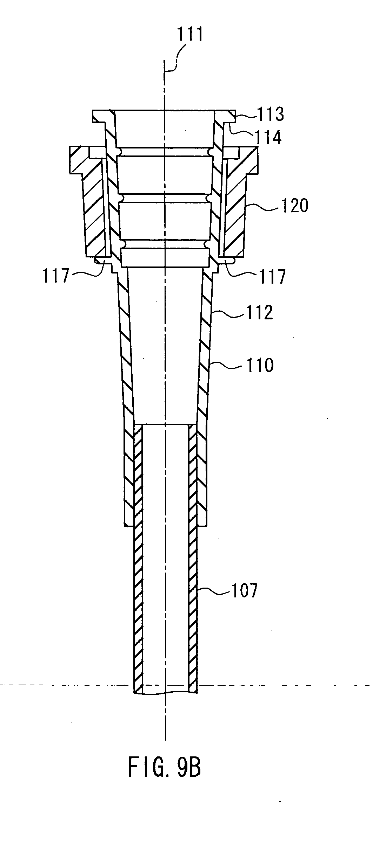

For example, as the mechanism holding the handle 120, as shown in FIG. 9A, a pair of locking protrusions 117 (in FIG. 9A, one of the locking protrusions 117 is not seen) protruding outward (in a direction orthogonal to the center axis 111) may be provided on the outer peripheral surface of the tube-shaped section 112 of the adaptor 110. As shown in FIG. 9B, the locking protrusions 117 prevent the handle 120 from dropping by abutting on the lower end of the handle 120. That is, the handle 120 has its movement in the direction of the center axis 111 with respect to the adaptor 110 limited by the flange section 113 and the pair of locking protrusions 117. The locking protrusions 117 can be molded integrally with the adaptor 110, using the same material as that for the adaptor 110. Thus, the locking protrusions 117 can be deformed elastically and allow the handle 120 to move beyond the locking protrusions 117 from one side to the other side with respect to the locking protrusions 117 in the direction of the center axis 111. Although the number of the locking protrusions 117 is two in the example shown in FIGS. 9A and 9B, the present invention is not limited thereto, and one or at least three locking protrusions 117 may be used. In the case where a plurality of locking protrusions 117 are provided, it is preferred that they are placed at an equal angular interval with respect to the center axis 111. It is preferred that the plurality of locking protrusions 117 are provided at the same position in the direction of the center axis 111. Further, the locking protrusions 117 may be continuous in an annular shape in the circumferential direction of the tube-shaped section 112.

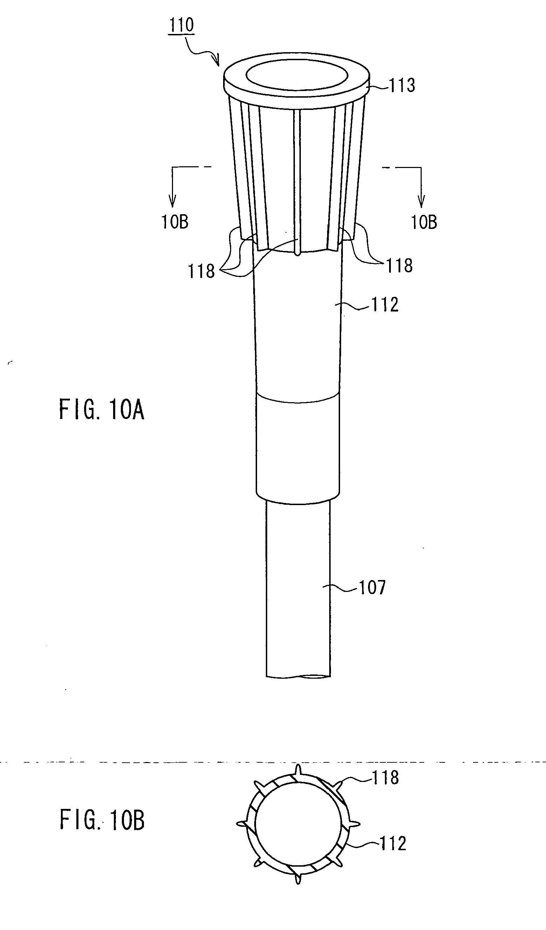

Alternatively, as the mechanism holding the handle 120, a plurality of contact protrusions 118 protruding outward (in a direction orthogonal to the center axis 111) may be provided on the outer peripheral surface of the adaptor 110, for example, as shown in FIGS. 10A and 10B. FIG. 10B is a cross-sectional view taken along a line 10B-10B in FIG. 10A. The plurality of contact protrusions 118 in the present example are formed in a ridge shape in an up-and-down direction at an equal angular interval with respect to the center axis 111 on the outer peripheral surface of the tube-shaped section 112 of the adaptor 120. The contact protrusions 118 can be molded integrally with the adaptor 110, using the same material as that for the adaptor 110. The plurality of contact protrusions 118 prevent the handle 120 from dropping by the static friction generated when the plurality of contact protrusions 118 come into contact with at least a part of the inner peripheral surface of the handle 120 (in particular, the inner peripheral surface of the small-diameter section 122) and are slightly deformed elastically. The shape and placement of the contact protrusions 118 are not limited to those in the example shown in FIGS. 10A and 10B. For example, the contact protrusions 118 may be any of protrusions in a ridge shape extending in the circumferential direction, protrusions in a ridge shape extending in a spiral shape, protrusions in a columnar shape or a cone shape, and the like.

Even in the case where the locking protrusions 117 shown in FIGS. 9A and 9B, and the contact protrusions 118 shown in FIGS. 10A and 10B are provided on the adaptor 110, the handle 120 can be moved to a position away from the adaptor 110. Thus, for example, in the case where it is difficult to insert the tubular section 172 deeply into the insertion section 119 since the outer diameter of the insertion section 119 is enlarged by the thick tubular section 172 and the outer peripheral surface of the insertion section 19 bumps against the inner peripheral surface of the handle 120, the handle 120 can be moved to a position where the handle 120 does not become an obstacle for inserting the tubular section 172 into the insertion section 119.

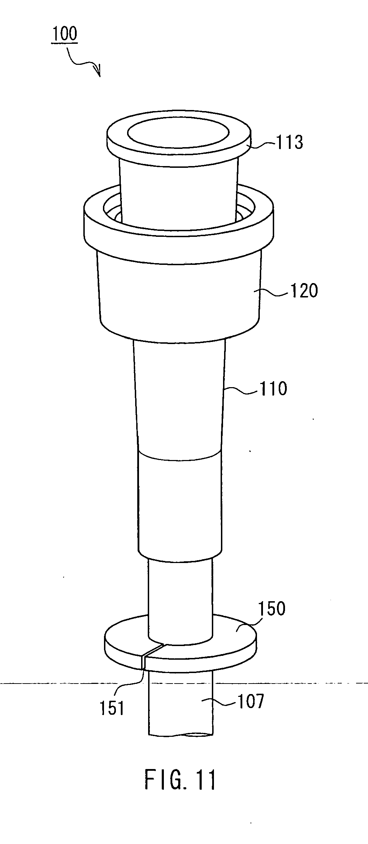

As the mechanism holding the handle 120, for example, as shown in FIG. 11, a holding member 150 may be attached to the tube 107 connected to the lower end of the adaptor 110. The holding member 150 is made of a material capable of being deformed elastically, and has a substantially doughnut shape. Since the inner diameter of the holding member 150 is the same as or slightly smaller than the outer diameter of the tube 107, the holding member 150 can be fixed to an arbitrary position of the tube 107 using friction. Since the outer diameter of the holding member 150 is larger than the inner diameter of the handle 120, the handle 120 cannot move beyond the holding member 150 to a position far away from the adaptor 110. The holding member 150 is provided with a slit 151 in a radial direction. The holding member 150 is deformed elastically so that two end faces of the holding member 150 sandwiching the slit 151 separate from each other in the up-and-down direction, whereby the position in the longitudinal direction of the tube 107 to which the holding member 150 is attached can be changed, and the holding member 150 can be attached/detached with respect to the tube 107 by allowing the tube 107 to pass through the slit 151. FIG. 11 shows an example, to which the present invention is not limited. For example, the holding member may be configured in such a manner that the tube 107 is sandwiched by two bar-shaped members placed in parallel. Further, the holding member may be bonded to the tube by a method such as adhesion or welding. Further, the holding member having a configuration similar to the above may be attached to the vicinity of the lower end of the adaptor 110, instead of the tube 107.

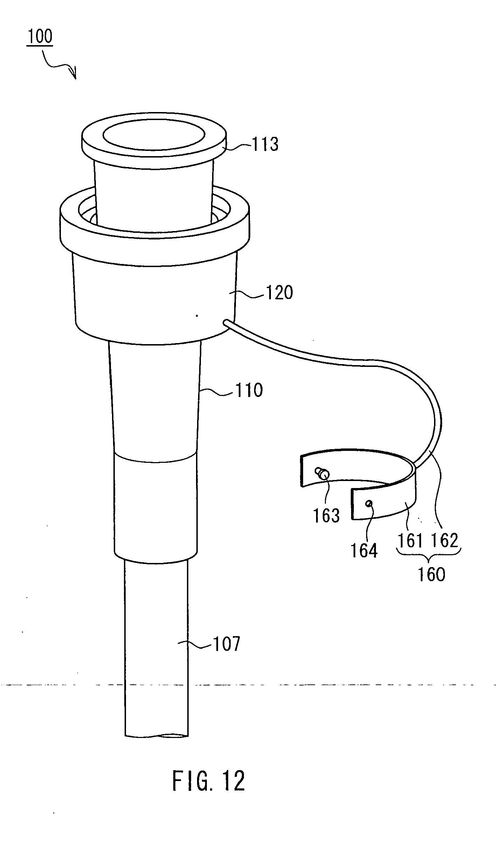

Alternatively, as the mechanism holding the handle 120, for example, as shown in FIG. 12, a holding member 160 may be attached to the handle 120. The holding member 160 is composed of a band 161 to be mounted on the tube 107, and a cord 162 connecting the band 161 to the handle 120. The band 161 has flexibility, and one end thereof is provided with a protrusion 163 and the other end thereof is provided with a through-hole 164. The distance between the protrusion 163 and the through-hole 164 is substantially the same as or shorter than the outer peripheral length of the tube 107. The band 161 is wound around the outer peripheral surface of the tube 107, and the protrusion 163 is inserted into the through-hole 164 to engage them with each other, whereby the band 161 is mounted so as to tighten the outer peripheral surface of the tube 107. In this state, the handle 120 cannot move to a position far away from the band 161 beyond the length of the cord 162. The band 161 may be mounted in the vicinity of the lower end of the adaptor 110, instead of the tube 107, by adjusting the length of the band 161 appropriately.

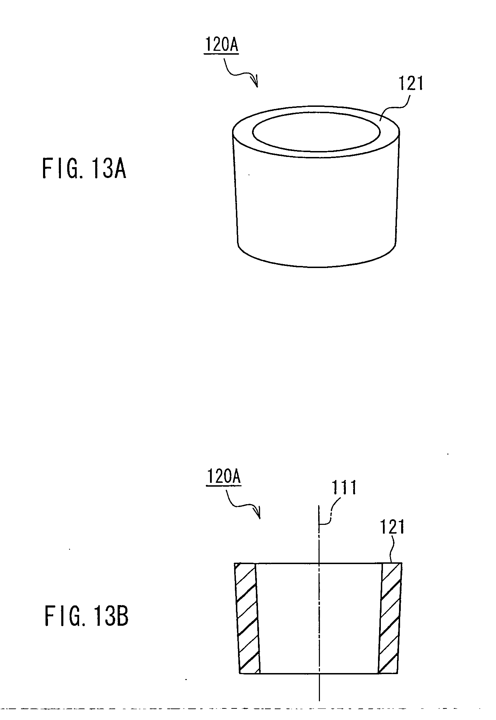

The handle 120 shown in FIGS. 3A and 3B has the large-diameter section 123 so as to store the flange section 113 of the adaptor 110. However, the handle of the present invention only needs to have the flange holding section 121 holding the lower surface 114 of the flange section 113 and may not have the large-diameter section 123. For example, as in a handle 120A shown in FIGS. 13A and 13B, the inner peripheral surface and the outer peripheral surface may have a tapered surface without a difference in level in a step shape,. In this case, the upper end face of the handle 120A functions as the flange holding section 121.

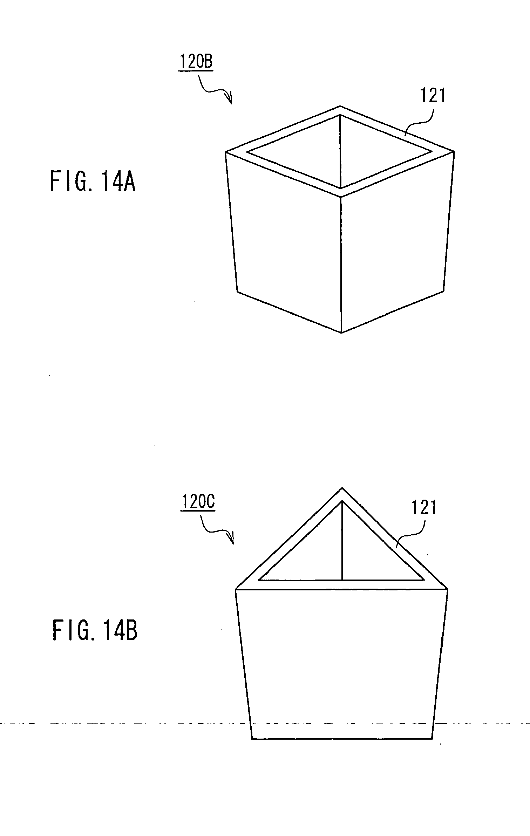

Further, the shape of the handle seen from above and below is not limited to a circle. For example, the shape seen from above and below may be a rectangle as in a handle 120B shown in FIG. 14A, a triangle as in a handle 120C shown in FIG. 14B, or other shapes such as a polygon, an oval, and an ellipse. Further, when seen from above and below, it is not necessary that the shape of the outer peripheral surface of the handle is matched with the shape of the inner peripheral surface thereof. For example, when seen from above and below, the inner peripheral surface surrounding the adaptor may be a circle and the outer peripheral surface may be a rectangle. Further, the inner peripheral surface of the handle may be provided with a plurality of protrusions in a dome shape, a cylindrical surface shape, or the like, or unevenness in a gear shape in the circumferential direction, and further, the inner peripheral surface of the handle may be provided with a minute uneven pattern.

Further, the inner size of the inner peripheral surface of the handle and/or the outer size of the outer peripheral surface thereof do not need to change in a tapered shape so that the upper end side is larger than the lower end side, and may be constant in the direction in the center axis 111. Alternatively, the inner size of the inner peripheral surface of the handle and/or the outer size of the outer peripheral surface thereof may change in a tapered shape so that the upper end side is smaller than the lower end side.

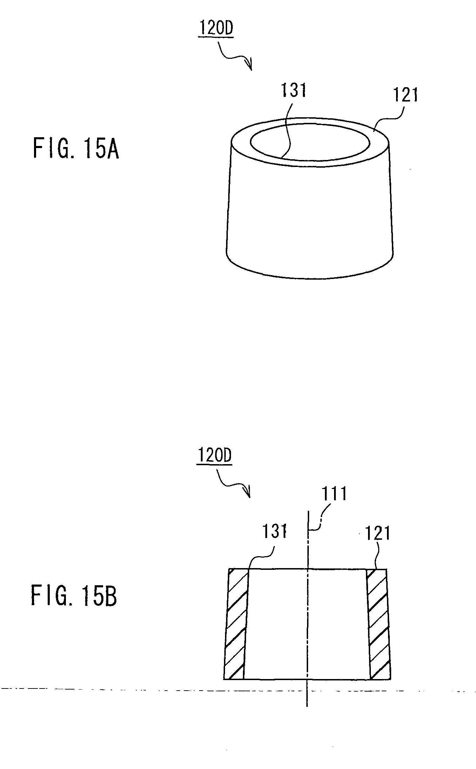

In particular, as in a handle 120D shown in FIGS. 15A and 15B, the inner size of the inner peripheral surface may change in a tapered shape so that the upper end side is smaller than the lower end side. As shown in FIG. 5A, the outer peripheral surface of the tubular section 172 of the male connector generally has a tapered surface so that the outer diameter decreases toward the tip end in most cases. When the handle 120D shown in FIGS. 15A and 15B is used with respect to the tubular section 172 of such a male connector, the inner diameter becomes minimum. An opening edge 131 on the upper end face locally comes into tight contact with the insertion section 119, in which the tubular section 172 is inserted, to fasten the insertion section 119. That is, a "squeezing effect" is obtained, in which the local part of the insertion section 119 is pinched strongly by the tubular section 172 on the inner side thereof and the opening edge 131 of the handle 120D on the outer side thereof. This enhances the connection strength between the tubular section 172 of the male connector and the adaptor 110. Thus, in the case of using the squeezing effect, the possibility that the adaptor 110 comes off the tubular section 172 accidentally is reduced and safety is enhanced, compared with the case of not using the squeezing effect.

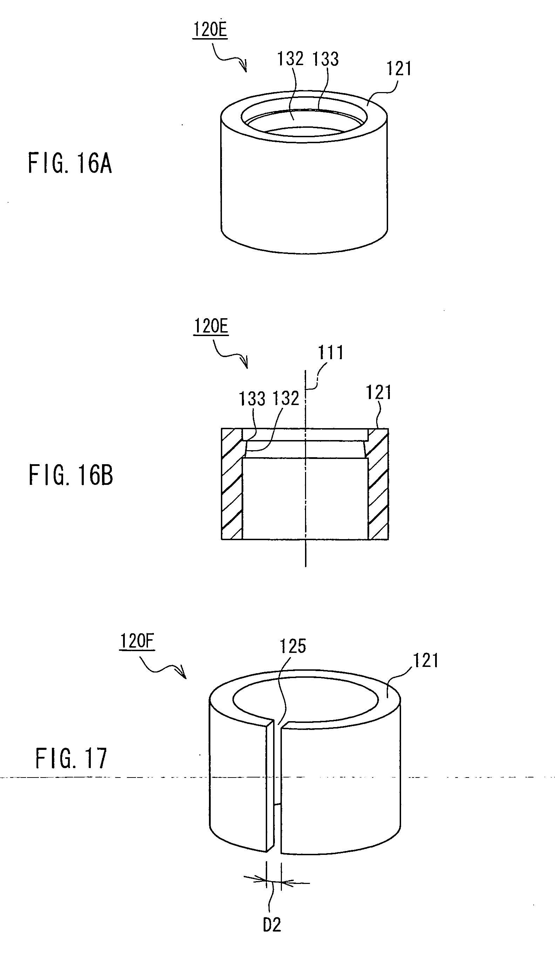

The shape of the handle exhibiting the above-mentioned squeezing effect is not limited to the handle 120D shown in FIGS. 15A and 15B. For example, as in a handle 120E shown in FIGS. 16A and 16B, a small-diameter section 132 whose inner size is smaller than that of the other portions may be formed at a position slightly away from the flange holding section 121 on the inner peripheral surface of the handle 120E. The small-diameter section 132 is formed in an annular shape continuously in the circumferential direction. An edge 133 on the upper side of the small diameter section 132 locally comes into tight contact with the insertion section 119, in which the tubular section 172 is inserted, to tighten the insertion section 119, thereby exhibiting the squeezing effect. In FIGS. 16A and 16B, the inner size of the inner peripheral surface of the small diameter Section 132 changes in a tapered shape so that the upper side is smaller than the lower side. This enables a larger squeezing effect to be obtained. It should be noted that the inner size of the inner peripheral surface of the small-diameter section 132 may be constant in the up-and-down direction.

In FIGS. 15A and 15B, and 16A and 16B, the position where the handle comes into tight contact with the outer peripheral surface of the insertion section 119 in which the tubular section 172 is inserted has an annular shape continuous in the circumferential direction. However, the present invention is not limited thereto, and the position where the handle comes into tight contact with the outer peripheral surface of the insertion section 119 may be discontinuous in the circumferential direction. For example, a plurality of protrusions in a dome shape, a cylindrical surface shape, or the like, or unevenness in a gear shape is formed in the circumferential direction at a position of the inner peripheral surface of the handle which is to come into tight contact with the outer peripheral surface of the insertion section 119, or the inner peripheral surface of the handle that comes into tight contact with the outer peripheral surface of the insertion section 119 is formed in a polygonal or oval shape, whereby the position where the insertion section 119 is squeezed may be made discontinuous.

Further, various uneven shapes or the like may be provided on the outer peripheral surface of the handle so as to enhance the grasping property and slip prevention for fingers.

Further, although the above-mentioned handles 120, 120A, 120B, 120C, 120D, and 120E all have an annular shape surrounding the adaptor 110 without a rift, the handle of the present invention is not limited thereto. For example, as in a handle 120F shown in FIG. 17, a slit 125 connecting the upper end to the lower end of the handle 120F may be formed so that the handle becomes discontinuous in the circumferential direction. The shape of the handle 120F seen from above and below is a substantially C-shape. This enables, for example, the following effect to be obtained. When the adaptor 110 is installed on the thick tubular section 172 with a handle, the insertion section 119 of the adaptor 110 elongates in accordance with the outer diameter of the tubular section 172 and the outer diameter of the insertion section 119 becomes large. If the handle is not provided with the slit 125, the upper limit of the outer diameter of the insertion section 119 is matched with the inner diameter of the inner peripheral surface of the handle that abuts on the outer peripheral surface of the adaptor. Thus, the upper limit of the outer diameter of the tubular section 172 capable of being connected to the adaptor 110 depends upon the inner diameter of the handle. In contrast, in the case of the handle 120F provided with the slit 125, the handle 120F is deformed elastically, whereby the inner diameter of the inner peripheral surface thereof can be enlarged. Thus, even in the handles having the same inner diameter, if the handle 120F provided with the slit 125 is used, the adaptor 110 can be connected to the thicker tubular section 172, compared with the handle not provided with the slit 125.

There is no particular limit to a width D2 of the slit 125 in the circumferential direction. If the width D2 is set to be narrow so that the adaptor 110 and the tube 107 connected thereto cannot pass through the slit 125, the handle 120F can be prevented from dropping. Conversely, for example, the width D2 can be set so large that the tube 107 can pass through the slit 125. By setting the width D2 as such, the handle 120F can be removed after the adaptor 110 is connected to the tubular section 172 of the male connector 170, using the handle 120F, and thereafter, the handle 120F can be attached again. That is, a female connector, with respect to which the handle 120F can be attached/detached, can be realized. For example, when the outer diameter of the insertion section 119 of the adaptor 110 is enlarged by the thick tubular section 172 of the male connector, and the elastic deformation of the handle 120F exceeds the upper limit of the allowable range, the handle 120F may be removed from the adaptor 110. Further, in the case where the handle does not have an engagement shape (see Embodiments 2 to 4 described later) adaptable to the male connector, the handle may be removed after the adaptor 110 and the tubular section 172 are connected to each other. Further, after that, in the case of connecting the adaptor to another tubular section, the handle can be fitted again.

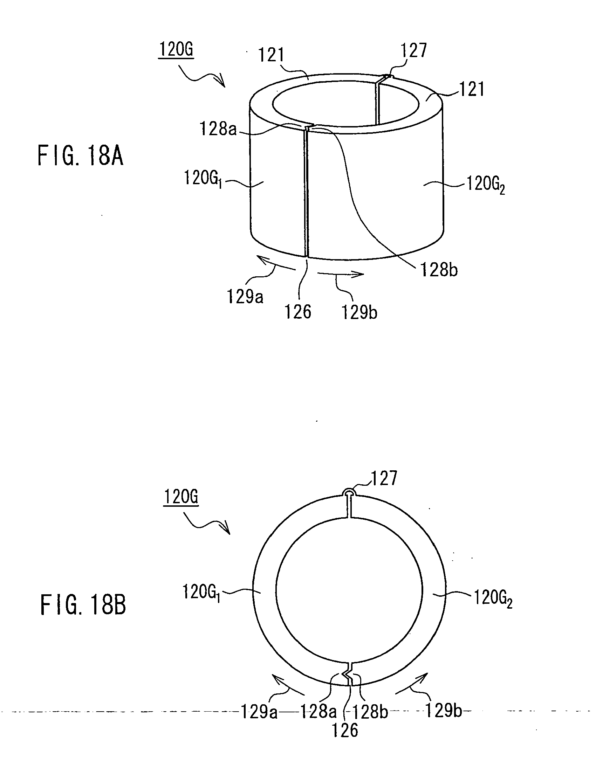

A handle 120G shown in FIGS. 18A and 18B is provided with a slit 126 connecting the upper end to the lower end of the handle 120G. A hinge 127 capable of being deformed elastically is formed at a position opposed to the slit 126 by setting the thickness relatively small. More specifically, the handle 120G has two halves 120G₁, 120G₂. The slit 126 is formed between each one end of the halves 120G₁, 120G₂, and the respective other ends are connected to each other via the hinge 127. On the end faces of the two halves 120G₁, 120G₂ opposed to each other via the slit 126, fitting shapes 128a, 128b to be fitted with each other are formed. The two halves 120G₁, 120G₂ can be rotated in directions indicated by arrows 129a, 129b with respect to the hinge 127 by deforming the hinge 127 elastically, which allows the end faces of the two halves 120G₁, 120G₂ opposed to each other via the slit 126 to separate from each other. The fitting shapes 128a, 128b prevent the positional shift between the end faces of the two halves 120G₁, 120G₂ when the end faces are brought into contact with each other. The handle 120G enables the adaptor 110 to be connected to the thick tubular section 172 in the same way as in the handle 120F. Further, a female connector, with respect to which the handle 120G can be attached/detached, can be realized.

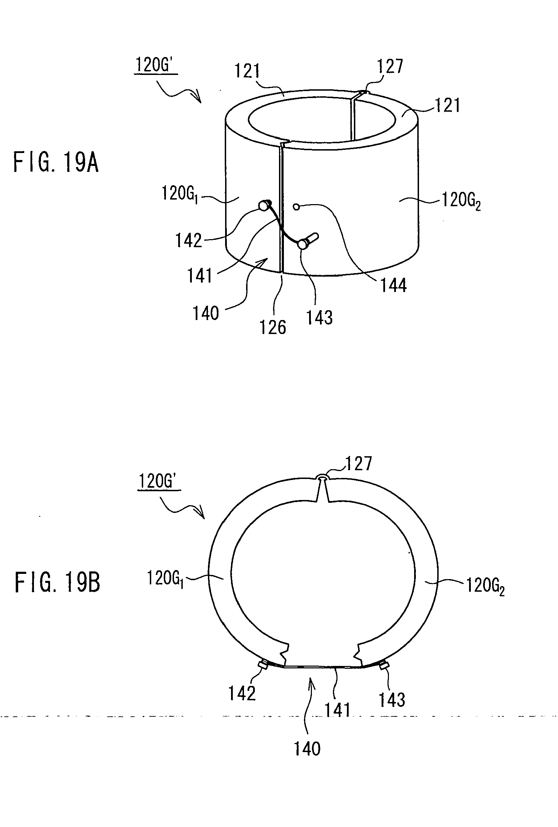

As shown in FIGS. 17, 18A, and 18B, when the handles 120F, 120G are provided with the slits 125, 126, the rigidity of the handles 120F, 120G decreases, so that the above-mentioned squeezing effect by the handle decreases. In order to obtain a desired squeezing effect even when a slit is formed, diameter expansion limitation means 140 that limits the expansion of the diameter of the handle may be provided as shown in FIGS. 19A and 19B. A handle 120G' shown in FIGS. 19A and 19B is the same as the handle 120G shown in FIGS. 18A and 18B except that the diameter expansion limitation means 140 is provided. As shown in FIG. 19A, the diameter expansion limitation means 140 includes a band 141 made of a material that is unlikely to elongate (i.e., that has a very high spring coefficient) and having flexibility. One end of the band 141 is fixed to the half 120G₁ with a fixing pin 142, and a locking pin 143 is attached to the other end of the band 141. A locking hole 144 is formed in the half 120G₂. In the case where the squeezing effect of the handle 120G' is required, the locking pin 143 is inserted into the locking hole 144, as shown in FIG. 19B. In the case where the outer diameter of the tubular section 172 is large, the hinge 127 is deformed elastically so that the two halves 120G₁, 120G₂ separate from each other and the diameter of the handle 120G' expands; however, the band 141 placed across the two halves 120G₁, 120G₂ limits the expansion of the diameter of the handle 120G'. This enables the squeezing effect to be obtained. It is preferred that the upper limit for the diameter of the handle 120G to expand varies depending upon the outer diameter of the tubular section 172, and for this purpose, for example, the length of the band 141 is changed, or a plurality of locking holes 144 are formed at different positions so as to change appropriately the locking hole 144 into which the locking pin 143 is inserted. FIGS. 19A and 19B show an example in which the diameter expansion limitation means 140 is provided at the handle 120G shown in FIGS. 18A and 18B; however, similar diameter expansion limitation means may be provided at the handle 120F shown in FIG. 17. The diameter expansion limitation means 140 shown in FIGS. 19A and 19B is an example, and the present invention is not limited to this configuration as long as the expansion of the diameter of the handle can be limited.

The movement limitation means of the female connector of the present invention sets at least the terminating end on the upper side (upper limit position) of the range in which the handle can move relative to the adaptor in the direction of the center axis 111 of the adaptor. As an example, in the above-mentioned female connector 100, the adaptor 110 is provided with the flange section 113, and the handle 120 is provided with the flange holding section 121 that abuts on the lower surface of the flange section 113 (FIG. 1).

However, the movement limitation means of the female connector of the present invention is not limited to the above, and further, the movement limitation means may set the terminating end on the lower side (lower limit position) of the range in which the handle can move relative to the adaptor in the direction of the center axis 111 of the adaptor.

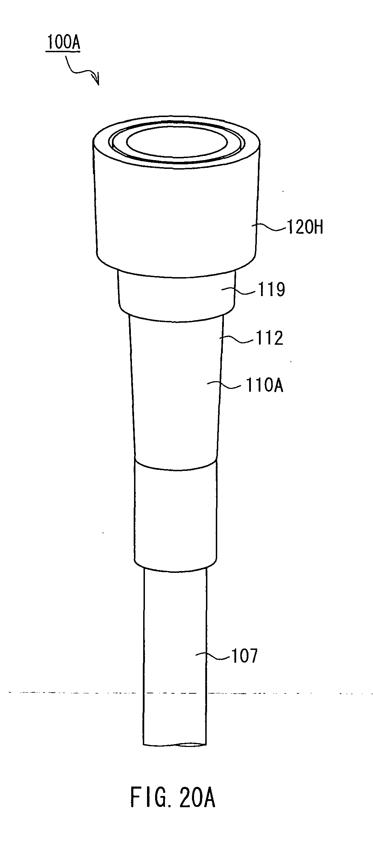

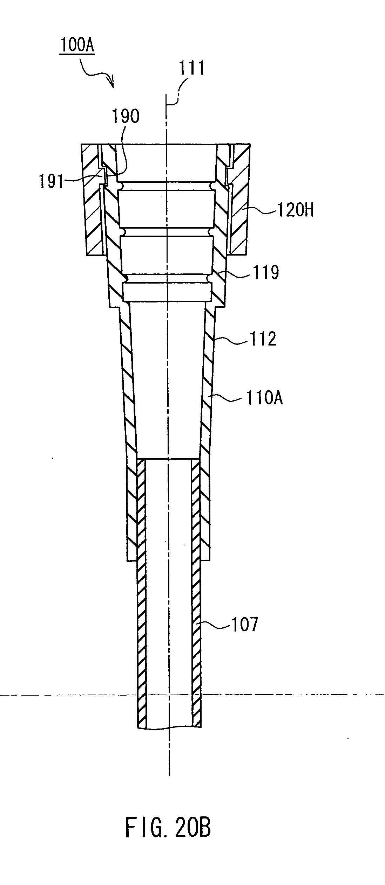

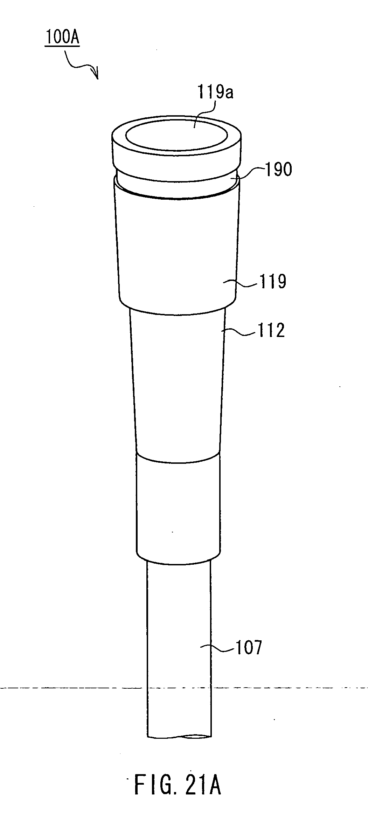

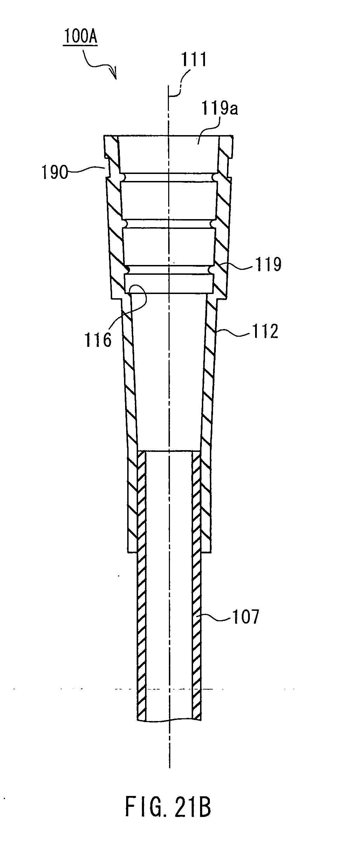

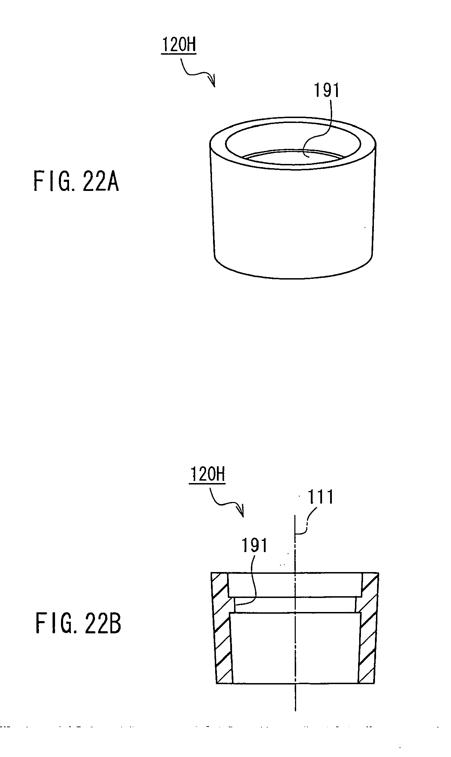

FIG. 20Ais a perspective view showing a schematic configuration of a female connector 100A according to Embodiment 1 having the movement limitation means that sets the terminating ends on the upper and lower sides of the range in which the handle moves with respect to the adaptor, and FIG. 20B is a cross-sectional view thereof. FIG. 21Ais a perspective view showing a schematic configuration of an adaptor 110A constituting the female connector 100A, and FIG. 21B is a cross-sectional view thereof. FIG. 22Ais a perspective view showing a schematic configuration of a handle 120H constituting the female connector 100A, and FIG. 22B is a cross-sectional view thereof. In these figures, members having the same functions as those shown in FIGS. 1A, 1B, 2A, 2B, 3A, and 3B are denoted with the same reference numerals as those therein, and the description thereof will be omitted.

The adaptor 110A shown in FIGS. 21A and 21B is different from the adaptor 110 shown in FIGS. 2A and 2B in that the flange section 113 is not provided and an annular groove 190 is formed, which is placed on the outer peripheral surface of the tube-shaped section 112 in the circumferential direction.

The handle 120H shown in FIGS. 22A and 22B is different from the handle 120 shown in FIGS. 3A and 3B in that the flange holding section 121 and the large-diameter section 123 are not provided, and an annular fitting section 191 is formed, which protrudes from the inner peripheral surface and is placed in the circumferential direction.

As shown in FIGS. 20A and 20B, the fitting section 191 of the handle 120H is fitted in the groove 190 of the adaptor 110A. As is apparent from them, in the female connector 100A, the groove 190 of the adaptor 110A and the fitting section 191 of the handle 120H function as the movement limitation means that sets the terminating ends on the upper and lower sides (upper and lower limit positions) of the range in which the handle 120H can move relative to the adaptor 110A in the direction of the center axis 111.

Further, the fitting section 191 provided on the handle 120H is allowed to exhibit the above-mentioned squeezing effect.

When the width of the groove 190 in the direction of the center axis 111 is defined to be W1 and the width of the fitting section 191 in the direction of the center axis is defined to be W2, it is preferred that 1.0 ≤ W1/W2 ≤ 1.5 is satisfied. When the W1/W2 is smaller than the lower limit, it is difficult to fit the fitting section 191 of the handle 120H in the groove 190 of the adaptor 110A. When W1/W2 is larger than the upper limit, the range in which the handle 120H can move relative to the adaptor 110A is enlarged. Therefore, the center axis of the handle 120H becomes likely to tilt with respect to the center axis 111 of the adaptor 110A, which makes it necessary to perform a complicated operation such as the correction of the direction of the tilting handle 120H or causes the fitting section 191 to come off the groove 190.

In the above-mentioned female connector 100A, a part of the inner peripheral surface of the handle 120H in the direction of the center axis 111 is protruded to form the fitting section 191; however, the entire inner peripheral surface of the handle 120H in the direction of the center axis 111 may be used as a fitting section. More specifically, the entire handle in the direction of the center axis 111 may be designed so as to be fitted in the groove 190 formed in the adaptor 110A

In the above-mentioned female connector 100A, the groove 190 is provided on the surface of the adaptor 110A opposed to the handle 120H, and the fitting section 191 is provided on the surface of the handle 120H opposed to the adaptor 110A. However, a groove may be provided on the surface of the handle 120H opposed to the adaptor 110A, and a fitting section to be fitted in the groove may be provided on the surface of the adaptor 110A opposed to the handle 120H.

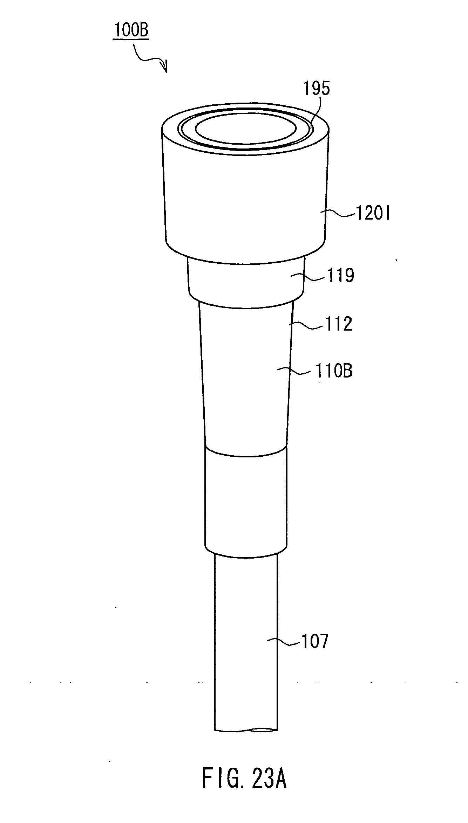

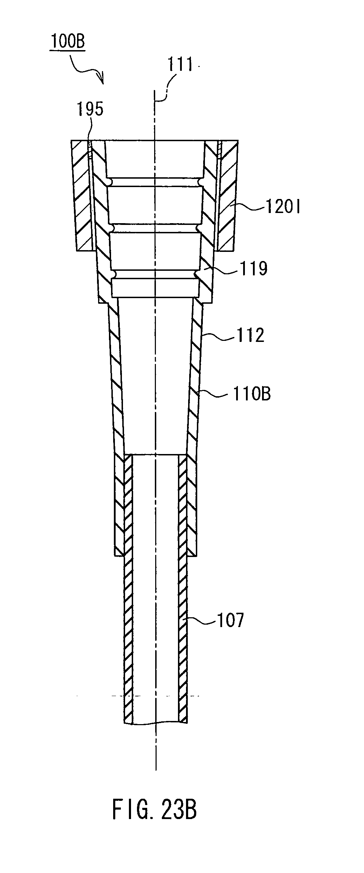

FIG. 23Ais a perspective view showing a schematic configuration of a female connector 100B according to Embodiment 1 having the movement limitation means that sets the terminating ends on the upper and lower sides of the range in which the handle moves with respect to the adaptor, and FIG. 23B is a cross-sectional view thereof. The female connector 100B includes an adaptor 110B and a handle 120I fitted on the adaptor 110B. In FIGS. 23A and 23B, members having the same functions as those in FIGS. 1A and 1B are denoted with the same reference numerals as those therein, and the description thereof will be omitted.

The female connector 100B shown in FIGS. 23A and 23B is different from the female connector 100 shown in FIGS. 1A and 1B in that the adaptor 110B does not have the flange section 113, the handle 120I does not have the flange holding section 121 and the large-diameter section 123, and the adaptor 110B and the handle 120I are bonded to each other by a bonding section 195. In the female connector 100B, the bonding section 195 functions as the movement limitation means that sets the terminating ends on the upper and lower sides (upper and lower limit positions) of the range in which the handle 120I can move relative to the adaptor 110B in the direction of the center axis 111.

A method for bonding the adaptor 110B and the handle 120I to each other by the bonding section 195 is not particularly limited, and for example, coating of an adhesive or welding can be used.

Various modified examples as described above can be applied to the above-mentioned female connectors 100A, 100B. The following should be noted: the movement range of the handles 120H, 120I with respect to the adaptors 110A, 110B is limited, so that the female connector having such movement limitation means does not require the mechanisms for holding a handle at a predetermined position shown in FIGS. 9 to 12.

### (Embodiment 2)

In Embodiment 2, a female connector in which an engagement shape for preventing an adaptor connected to a tubular section of a male connector from coming off the tubular section accidentally is provided on a handle, and a male connector provided with an engagement shape corresponding thereto will be described.

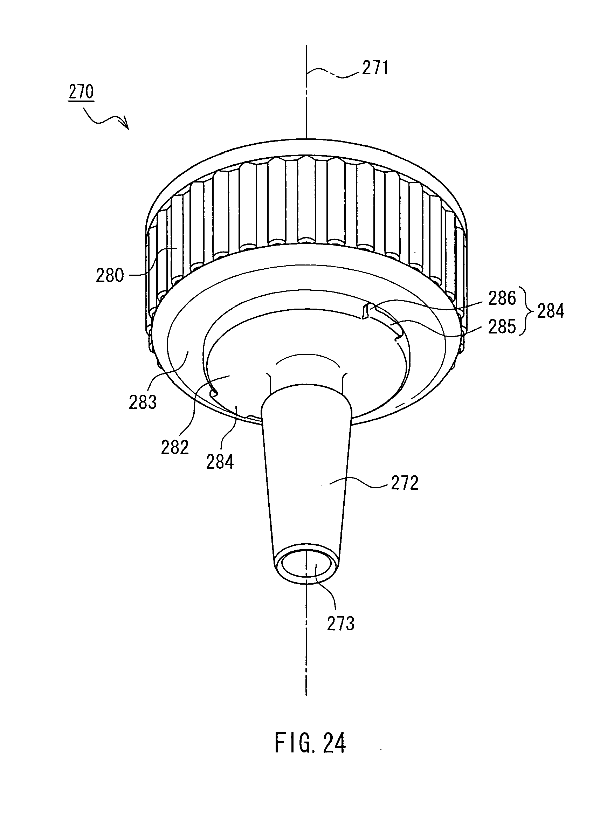

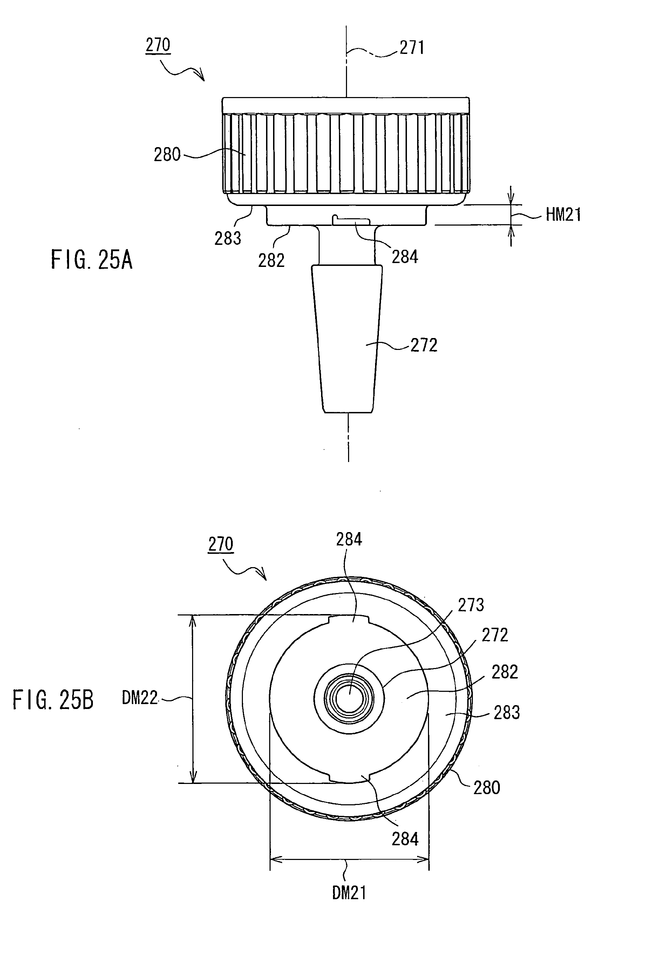

FIG. 24 is a perspective view of a male connector 270 according to Embodiment 2 of the present invention. FIG. 25Ais a front view of the male connector 270, and FIG. 25B is a bottom view thereof. In FIG. 24, alternate long and short dashed lines 271 indicate a center axis of the male connector 270. The direction of the center axis 271 is defined as an up-and-down direction, and an upper side on the drawing surface of FIG. 24 (a side connected to the port 930 of the medical container 910) will be referred to as an "upper side", and a lower side on the drawing surface of the figure (a side to which a female connector is connected) will be referred to as a "lower side".

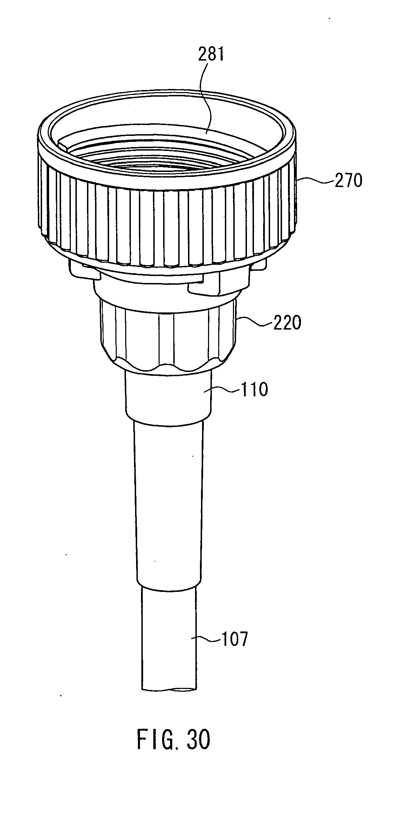

On the inner peripheral surface of a cap section 280, a female thread 281 (see FIG. 30) to be screwed with the male thread section 936 (see FIG. 4) of the port 930 of the medical container 910 is formed. On the lower surface of the cap section 280, a circular region protrudes downward by a height HM21 from the center compared with an annular region 283 in the surrounding of the circular region, whereby a platform 282 is provided. A tubular section 2 72 protruding downward is provided at the center of the platform 282. The outer peripheral surface of the tubular section 272 of the present example has a tapered surface (truncated cone surface) with an outer diameter increasing toward the cap 280. In the tubular section 272, a through-hole 273, out of which the liquid-like substance in the pouch 920 flows, is formed.

On the outer peripheral surface of the platform 282, a pair of engagement tabs 284 are formed so as to protrude in a radial direction with respect to the center axis 271 at symmetrical positions with respect to the center axis 271. The engagement tab 284 includes an engagement chip 285 extending substantially in a horizontal direction, and a lock protrusion 286 formed so as to protrude upward at one end of the engagement chip 285. The lower surface of the platform 282 is a flat surface, and is matched with the lower surfaces of the pair of engagement tabs 284. The shapes of the pair of engagement tabs 284 are symmetrical with respect to the center axis 271. The outer diameter of the platform 282 is DM21, and the distance between tops of the pair of engagement tabs 284 is DM22 (DM22 > DM21).

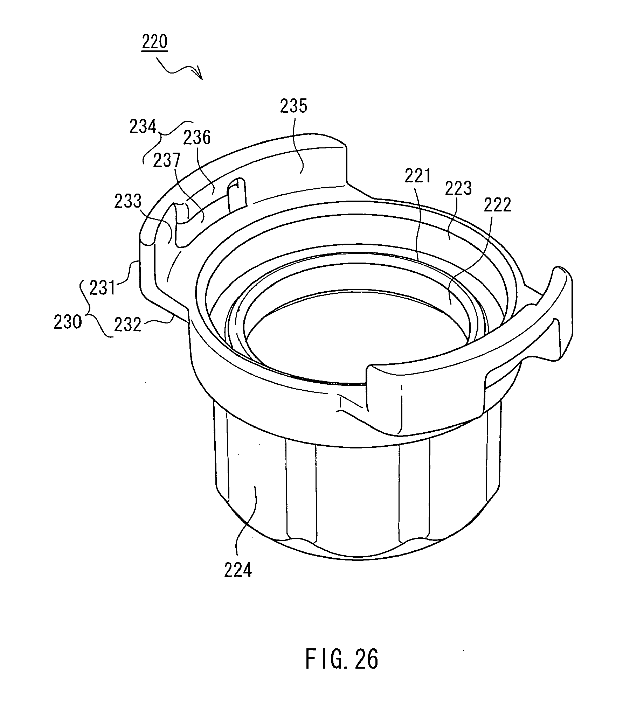

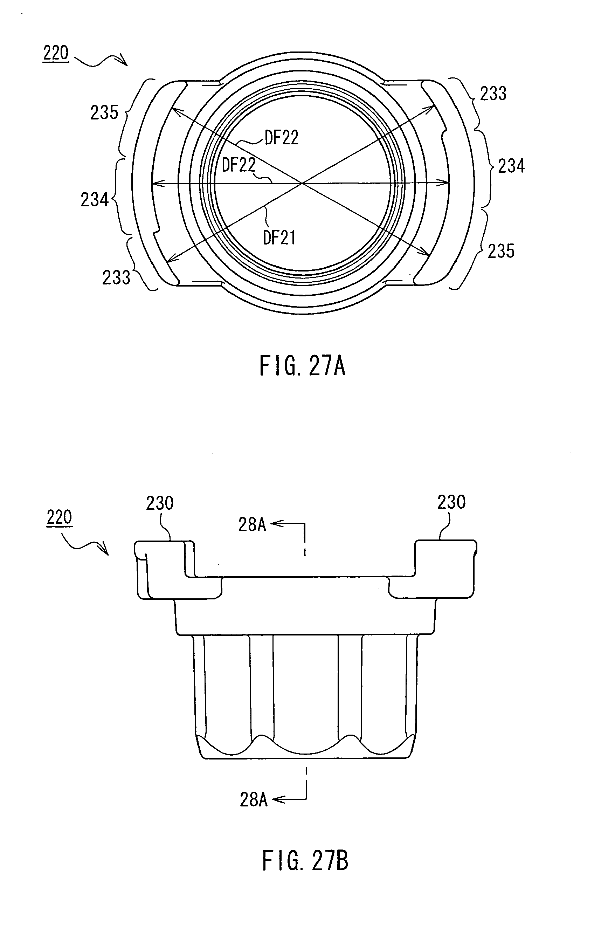

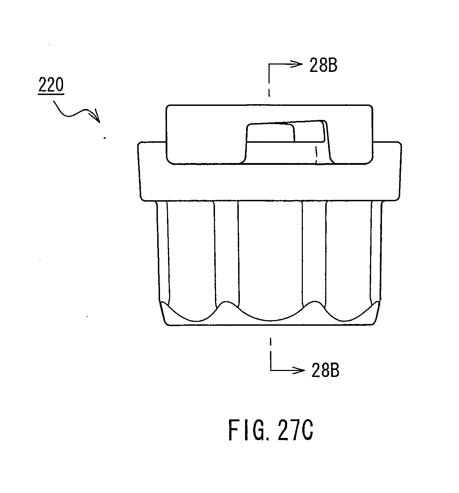

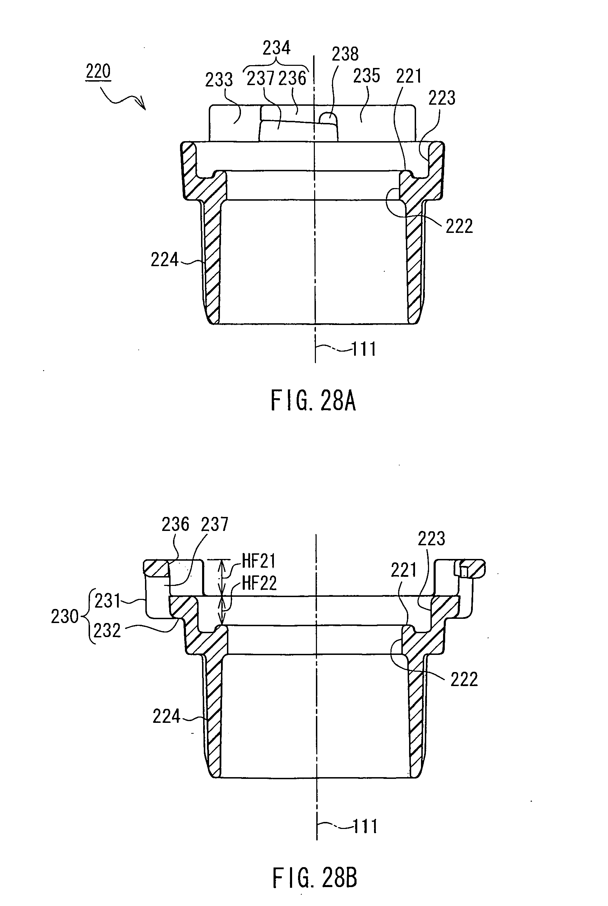

FIG. 26 is a perspective view of a handle 220 constituting the female connector according to Embodiment 2. FIG. 27Ais a plan view of the handle 220, FIG. 27B is a front view thereof, and FIG. 27C is a right side view thereof. FIG. 28A is a cross-sectional view taken along a line 28A-28A in FIG. 27B, and FIG. 28B is a cross-sectional view taken along a line 28B-28B in FIG. 27C. Since an adaptor constituting the female connector according to Embodiment 2 together with the handle 220 is the same as that described in Embodiment 1, the description thereof will be omitted. In FIGS. 28A and 28B, alternate long and short dashed lines 111 indicate the center axis of the adaptor described in Embodiment 1 to be inserted into the handle 220, and the center axis of the adaptor is matched with the center axis of the female connector of Embodiment 2 and the center axis of the handle 220. As described in Embodiment 1, the direction of the center axis 111 is defined as an up-and-down direction, and a side connected to the male connector will be referred to as an "upper side", and an opposite side thereof will be referred to as a "lower side".

The handle 220 has a substantially cylindrical shape as a whole, and has a large-diameter section 223 having an inner diameter larger than the outer diameter of the flange section 113 of the adaptor 110 in the vicinity of the upper end thereof, and a small-diameter section 222 having an inner diameter larger than the outer diameter of the tube-shaped section 112 of the adaptor 110 and smaller than the outer diameter of the flange section 113 on the lower side of the large-diameter section 223. Then, a flange holding section 221 is formed between the small-diameter section 222 and the large-diameter section 223. A grasping section 224 in a substantially cylindrical shape is provided on the lower side of the small-diameter section 222.

Apair of brim sections 230 are formed around and on the upper side of the large-diameter section 223. The brim section 230 includes an arc-shaped wall 231 substantially along the cylindrical surface with the center axis 111 being a center axis, and a bridge section 232 that extends in a direction perpendicular to the center axis 111 and connects the lower end of the arc-shaped wall 231 to the upper end of the large-diameter section 223. The arc-shaped wall 231 roughly is classified into three regions: a passage region 233, an engagement region 234, and a sliding region 235, in accordance with the difference in shape of the inner peripheral surface opposed to the center axis 111. The inner peripheral surface of the passage region 233 is apart of the cylindrical surface with a diameter DF21, and the inner peripheral surface of the sliding region 235 is a part of the cylindrical surface with a diameter DF22 (DF22 < DF21). The engagement region 234 between the passage region 233 and the sliding region 235 includes an engagement wall 236 at an upper end, which extends in the circumferential direction so as to connect the upper end of the passage region 233 to the upper end of the sliding region 235. A region between the engagement wall 236 and the bridge section 232 is dented in a concave shape, and in the present example, a through-hole 237 opened in the radial direction is formed. The inner peripheral surface of the engagement wall 236 opposed to the center axis 111 is a p art of the cylindrical surface with a diameter DF22. Thus, when seen from the center axis 111, the through-hole 237 provided on the bridge section 232 side with respect to the engagement wall 236 is recessed relatively to form a concave portion, and the sliding region 235 adjacent to the through-hole 237 in the circumferential direction protrudes relative to the through-hole 237 to form a convex portion. On the lower surface (the surface on the bridge section 232 side) of an end of the engagement wall 236 on the sliding region 235 side is provided with a lock concave portion 238 dented in a concave shape. The height of the arc-shaped wall 231 from the upper surface of the bridge section 232 in the direction of the center axis 111 is HF21. The pair of brim sections 230 are symmetrical with respect to the center axis 111. A distance HF22 in the direction of the center axis 111 between the upper surface of the flange holding section 221 and the upper surface of the bridge section 232 is substantially the same as or slightly larger than the distance in the direction of the center axis 111 from the upper end of the adaptor 110 to the lower surface 114 of the flange section 113.

The inner size DF21 between a pair of opposed passage regions 233 is larger than the distance DM22 between the tops of the pair of engagement tabs 284 of the male connector 270 (DM22 < DF21). The inner size DF22 between a pair of opposed sliding regions 235 is smaller than the distance DM22 between the tops of the pair of engagement tabs 284 of the male connector 270 and larger than the outer diameter DM21 of the platform 282 (DM21 < DF22 < DM22). The height HF21 of the arc-shaped wall 231 is substantially the same as the height HM21 of the platform 282 of the male connector 270 (HF21 ≈ HM21).

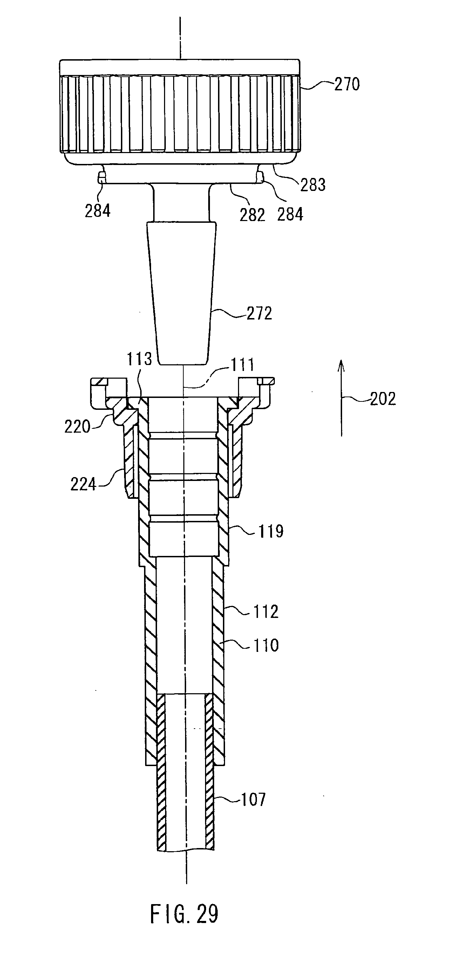

A method for connecting the female connector to the male connector 270 in Embodiment 2 will be described with reference to FIG. 29. In FIG. 29, a medical container including a port, to which the male connector 270 is connected, is omitted The adaptor 110 is inserted into the handle 220, and the lower surface 114 of the flange section 113 of the adaptor 110 is supported by the flange holding section 221 of the handle 220. In this state, the outer peripheral surface of the grasping section 224 of the handle 220 is grasped with two fingers, and the adaptor 110 is installed on the tubular section 272 in the direction indicated by an arrow 202 so that the adaptor 110 covers the outer peripheral surface of the tubular section 272 of the male connector 270. In the same way as in Embodiment 1, the insertion section 119 of the adaptor 110 has flexibility and elasticity, so that the insertion section 119 is deformed in accordance with the shape of the outer peripheral surface of the tubular section 272 of the male connector 270 and comes into tight contact with the outer peripheral surface of the tubular section 272 due to the elastic recovery force. On the other hand, the handle 220 has a high rigidity, so that the grasping force applied to the handle 220 in a diameter direction thereof by the two fingers will not increase the friction between the insertion section 119 of the adaptor 110 and the tubular section 272. Further, the flange section 113 is formed at a front end of the adaptor 110 in the direction indicated by the arrow 202, so that the adaptor 110 will not be buckled and deformed due to the force applied to the handle 220 in the direction indicated by the arrow 202 by the two fingers.

After the tubular section 272 of the male connector 270 is inserted deeply into the insertion section 119 of the adaptor 110, the handle 220 further is pushed in the direction indicated by the arrow 202 with respect to the male connector 270. The flange section 113 of the adaptor 110 is deformed elastically in the direction indicated by the arrow 202 by the flange holding section 221 of the handle 220, and the outer peripheral surface of the platform 282 of the male connector 270 is fitted between the pair of sliding regions 235 of the arc-shaped walls 231 of the handle 220. Finally, the upper end faces of the arc-shaped walls of the handle 220 abut on the annular region 283 of the male connector 270, and the upper surfaces of the bridge sections 232 of the handle 220 abut on the lower surface of the platform 282 of the male connector 270. In this state, the handle 220 is rotated clockwise around the center axis 111 with respect to the male connector 270. The pair of engagement tabs 284 formed on the outer peripheral surface of the platform 282 of the male connector 270 pass through the pair of passage regions 233 of the arc-shaped walls 231 of the handle 220 to reach the engagement regions 234. The inner size DF22 between the pair of sliding regions 235 is smaller than the distance DM22 between the tops of the pair of engagement tabs 284 of the male connector 270. Therefore, after the engagement tabs 284 abut on the ends of the sliding regions 235 on the engagement region 234 side, the handle 220 cannot be rotated any more with respect to the male connector 270. When the hand is taken off the handle 220 in this state, the handle 220 moves slightly in the direction opposite to the arrow 202 due to the elastic recovery force of the flange section 113 of the adaptor 111 that is deformed elastically in the direction indicated by the arrow 202, the lock protrusions 286 of the engagement tabs 284 of the male connector 270 are fitted in the lock concave portions 238 of the handle 220, and the engagement chips 285 of the engagement tabs 284 of the male connector 270 and the engagement walls 236 of the handle 220 are engaged with each other. Thus, the handle 220 and the male connector 270 are engaged with each other, whereby the adaptor 110 can be prevented from coming off the tubular section 272. FIG. 30 shows a state in which the female connector and the male connector 270 are connected to each other. Since the lock protrusions 286 of the engagement tabs 284 of the male connector 270 are fitted in the lock concave portions 238 of the handle 220, even when a small impact such as the touch of a hand is applied, the engagement state between the engagement chips 285 of the engagement tabs 284 of the male connector 270 and the engagement walls 236 of the handle 220 will not be broken.

The female connector can be separated from the male connector 270 by performing the above operation in an opposite way. That is, while the handle 220 is being pressed in the direction indicated by the arrow 202 with respect to the male connector 270, the handle 220 is rotated counterclockwise around the center axis 111 with respect to the male connector 270. Consequently, the lock protrusions 286 of the engagement tabs 284 of the male connector 270 come off the lock concave portions 238 of the handle 220, and the engagement tabs 284 of the male connector 270 move into the passage regions 233 of the handle 220. The handle 220 moves slightly in the direction opposite to the arrow 202 due to the elastic recovery force of the flange section 113 of the adaptor 110 that is deformed elastically in the direction indicated by the arrow 202, whereby the engagement between the engagement chips 285 of the engagement tabs 284 and the engagement walls 236 of the engagement regions 234 is cancelled. Then, the adaptor 110 is pulled out from the tubular section 272 of the male connector 270.

If the shape of the periphery of the engagement tab 284 including the platform 282, provided on the male connector 270, corresponds to the shape of the brim section 230 provided on the handle 220, the handle 220 and the male connector 270 can be engaged with each other. That is, even if the shape, size, and the like of the tubular section 272 of the male connector 270 vary depending upon the manufacturers and specifications, the handle 220 and the male connector 270 can be engaged with each other.

On the other hand, in Embodiment 2, in the same way as in Embodiment 1, even if the shape, size (for example, a taper angle, an outer diameter, etc.), and the like of the outer peripheral surface of the tubular section 272 of the male connector 270 vary depending upon the manufacturers and specifications, the insertion section 119 of the adaptor 110 is deformed elastically in accordance with the outer peripheral surface of the tubular section 272 of the male connector 270, so that the male connector 270 and the female connector 200 can be connected to each other precisely without the leakage of the liquid-like substance.

Thus, in the case where the male connector has the engagement tab 284 corresponding to the shape of the brim section 230 of the handle 220 in Embodiment 2, the adaptor 110 can be connected to the tubular section of the male connector, using the handle 220, and further, the handle 220 and the male connector can be engaged with each other.

On the other hand, in the case where the male connector does not have the engagement tab 284 corresponding to the shape of the brim section 230 of the handle 220 in Embodiment 2, the handle 220 is used only for connecting the adaptor 110 to the tubular section of the male connector. In this case, it is preferred that the mechanism (see FIGS. 9A, 9B, 10A, and 10B) holding the handle 220, described in Embodiment 1, is provided on the adaptor 110 so that the handle 220 does not move to a position far away from the adaptor 110 after the adaptor 110 is connected to the tubular section of the male connector.

According to Embodiment 2, the handle 220 and the male connector 270 can be engaged with each other, and the engagement can be cancelled merely by rotating the handle 220 around the center axis 111 with respect to the male connector 270. Further, the rotation angle required at this time is small, i.e., less than 180° in the above example, and thus, the operability is satisfactory.

Further, the height HF21 of the arc-shaped wall 231 is substantially the same as the height HM21 of the platform 282 of the male connector 270 (HF21 ≈ HM21): Thus, when the upper end face of the arc-shaped wall 231 of the handle 220 abuts on the annular region 283 of the male connector 270, the upper surface of the bridge section 232 of the handle 220 simultaneously abuts on the lower surface of the platform 282 of the male connector 270. Consequently, the handle 220 and the male connector 270 can be engaged with each other precisely. Further, after the engagement, the gap between the arc-shaped wall 231 of the handle 220 and the annular region 283 of the male connector 270 can be reduced, so that the connecting portion between the adaptor 110 and the tubular section 272 of the male connector 270 can be substantially covered with the arc-shaped wall 231, which is advantageous from the sanitary point of view.

Further, since the lower surface of the platform 282 of the male connector 270 is matched with the lower surface of the engagement tab 284 (that is, the engagement tab 284 is provided along the lower surface of the platform 282), the height HM21 of the platform 282 of the male connector 270 can be reduced Thus, the height HF21 of the arc-shaped wall 231 of the handle 220 also can be reduced. This can reduce the size in an up-and-down direction of the male connector 270 and the handle 220.

The above-mentioned embodiment is an example, and the present invention is not limited thereto.

For example, in the above embodiment, although the region between the engagement wall 236 and the bridge section 232 of the engagement region 234 of the handle 220 is the through-hole 237, the through-hole 237 may be dosed with a wall. This enables the connecting portion between the adaptor 110 and the tubular section 272 of the male connector 270 to be covered with the arc-shaped wall 231 of the brim section 230, which is advantageous from the sanitary point of view. At this time, the inner peripheral surface of the wall needs to be recessed from the inner peripheral surface of the engagement wall 236 so as to store the engagement tab 284, and for example, the inner peripheral surface of the wall may be a part of the cylindrical surface with the diameter DF21 that is the same as that of the inner peripheral surface of the passage region 233.

Although the height HF21 of the arc-shaped wall 231 and the height HM21 of the platform 282 of the male connector 270 are substantially the same (BF21 ≈ HM21), both the heights may be different from each other.

Further, the lower surface of the platform 282 of the male connector 270 and the lower surface of the engagement tab 284 may not be matched with each other.

Although the lock concave portion 238 is provided at an end of the engagement wall 236 on the sliding region 235 side, the lock concave portion 238 only needs to be provided on the engagement wall 236 without being limited to the above position. Similarly, the lock protrusion 286 is provided at one end of the engagement chip 285; however, the lock protrusion 286 only needs to be provided at the engagement chip 285 without being limited to the above position.

Although the platform 282 of the male connector 270 has two engagement tabs 284, the number of the engagement tabs 284 may be three or more, instead of being limited to two. It is preferred that the plurality of engagement tabs 284 are placed at an equal angular interval with respect to the center axis 271 of the male connector 270, irrespective of the number of the engagement tabs 284. Similarly, the number of the brim sections 230 of the handle 220 may be three or more, instead of being limited to two.

Further, in the above embodiment, although the two brim sections 230 are formed discontinuously around the handle 220, the brim sections 230 may be formed in an annular shape continuously around the handle 220. Even in this case, the engagement region 234 is placed considering the arrangement of the engagement tab 284 of the male connector 270, and the passage region 233 and the sliding region 235 are placed with the engagement region 234 interposed therebetween. The operation of engaging the handle 220 and the male connector 270 with each other is facilitated by enlarging the length of the passage region 233 in the circumferential direction. The connecting portion between the adaptor 110 and the tubular section 272 of the male connector 270 can be covered substantially with the arc-shaped wall 231 of the brim section 230 by forming the brim section 230 in an annular shape, which is advantageous from a sanitary point of view.

In the present embodiment, the description of Embodiment 1 can be adapted as it is or with obvious alterations added thereto appropriately.

### (Embodiment 3)

In Embodiment 3, a female connector in which an engagement shape for preventing an adaptor connected to a tubular section of a male connector from coming off the tubular section accidentally is provided on a handle, and a male connector provided with an engagement shape corresponding thereto will be described.

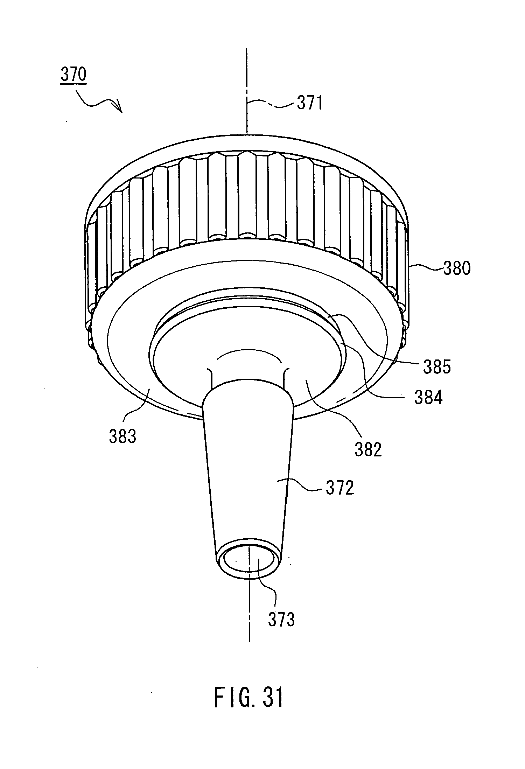

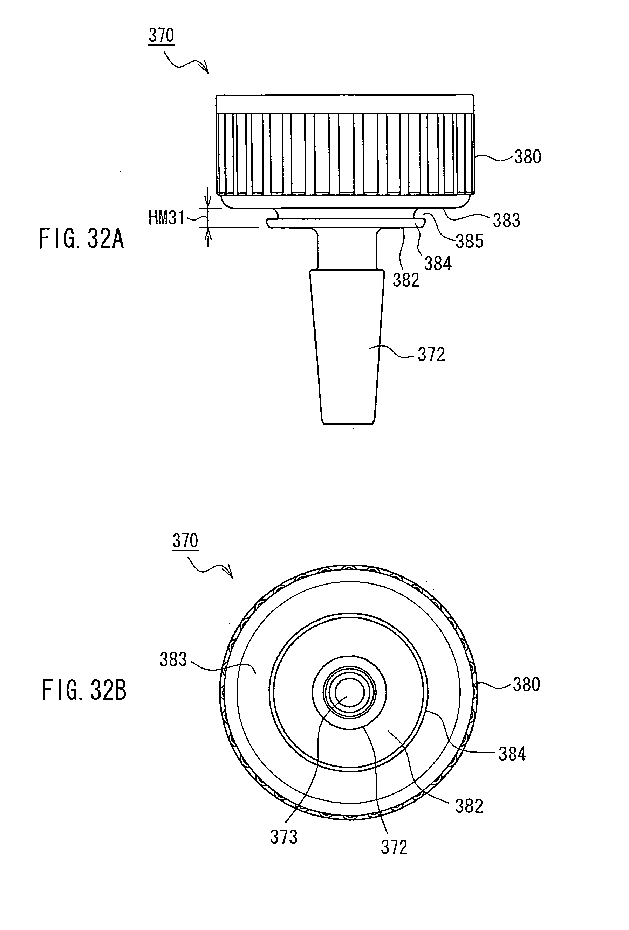

FIG. 31 is a perspective view of a male connector 370 according to Embodiment 3 of the present invention. FIG. 32Ais a front view of the male connector 370, and FIG. 32B is a bottom view thereof. In FIG. 31, alternate long and short dashed lines 371 indicate a center axis of the male connector 370. The direction of the center axis 371 is defined as an up-and-down direction, and an upper side on the drawing surface of FIG. 32 (a side connected to the port 930 of the medical container 910) will be referred to as an "upper side" and a lower side on the drawing surface of the figure (a side connected to a female connector) will be referred to as a "lower side".

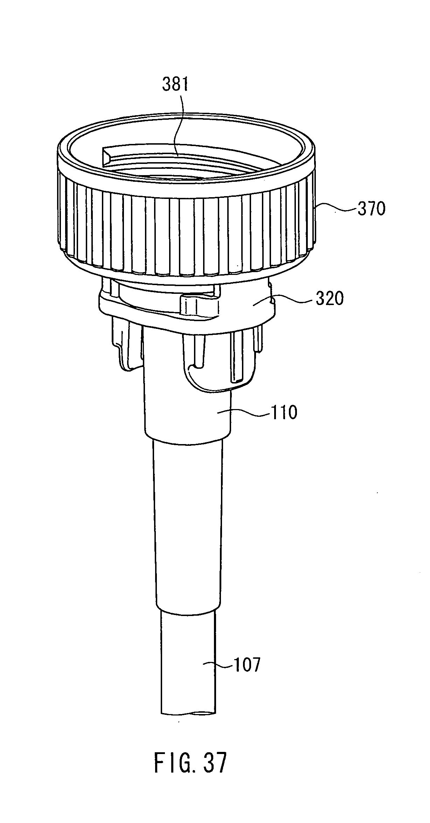

On the inner peripheral surface of a cap section 380, a female thread 381 to be screwed with the male thread section 936 (see FIG. 4) of the port 930 of the medical container 910 is formed (see FIG. 37). On the lower surface of the cap section 380, a circular region protrudes downward from the center compared with an annular region 383 in the surrounding of the circular region, whereby a platform 382 is provided. A tubular section 372 protruding downward is provided at the center of the platform 382. The outer peripheral surface of the tubular section 372 of the present example has a tapered surface (truncated cone surface) with an outer diameter increasing toward the cap 380. In the tubular section 372, a through-hole 373, out of which the liquid-like substance in the pouch 920 flows, is formed.

On the outer peripheral surface of the platform 382 , an engagement protrusion 384 protruding in the radial direction with respect to the center axis 371 is formed in an annular shape continuously over the entire periphery of the platform 382. The lower surface of the platform 382 is a flat surface, and is matched with the lower surface of the engagement protrusion 384. An undercut section 385 with an outer diameter smaller than that of the engagement protrusion 384 is formed between the engagement protrusion 384 and the annular region 383.

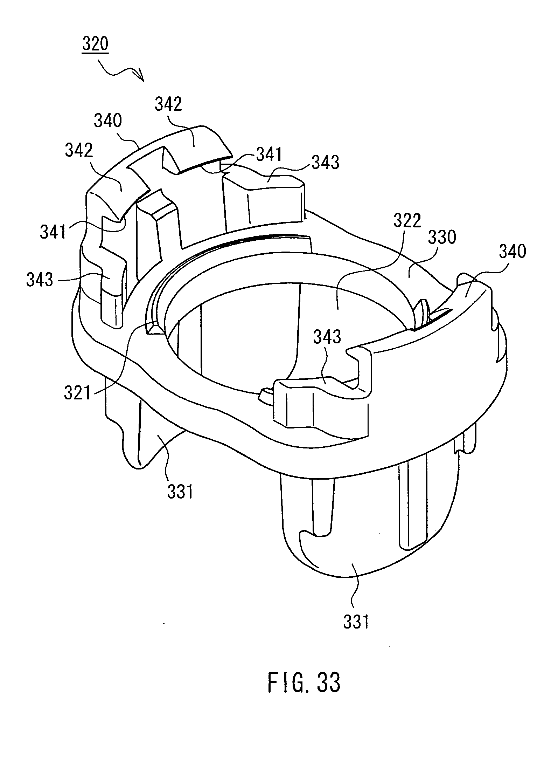

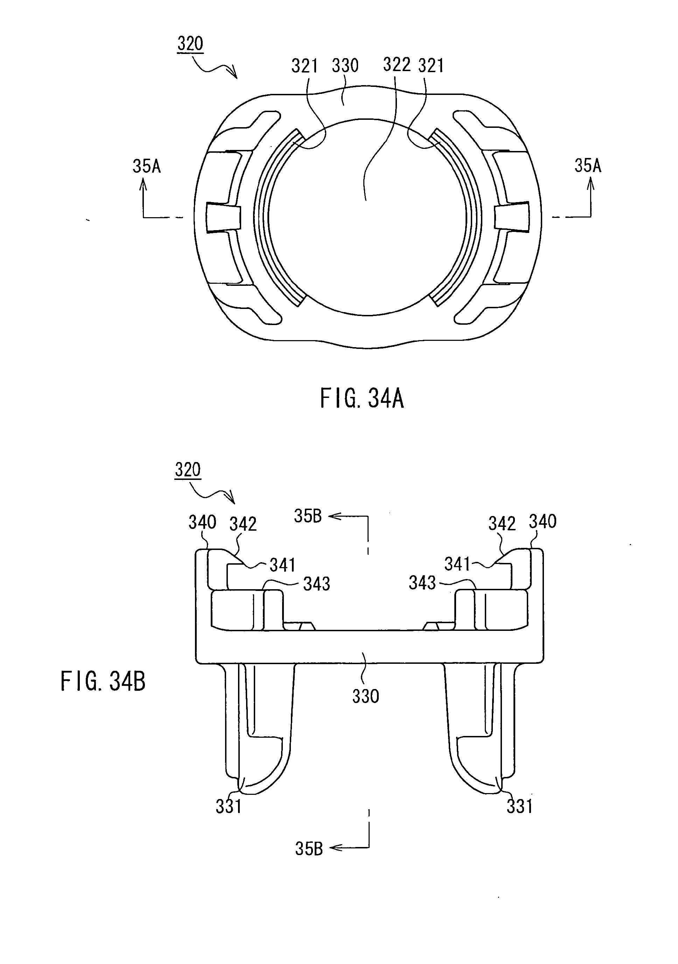

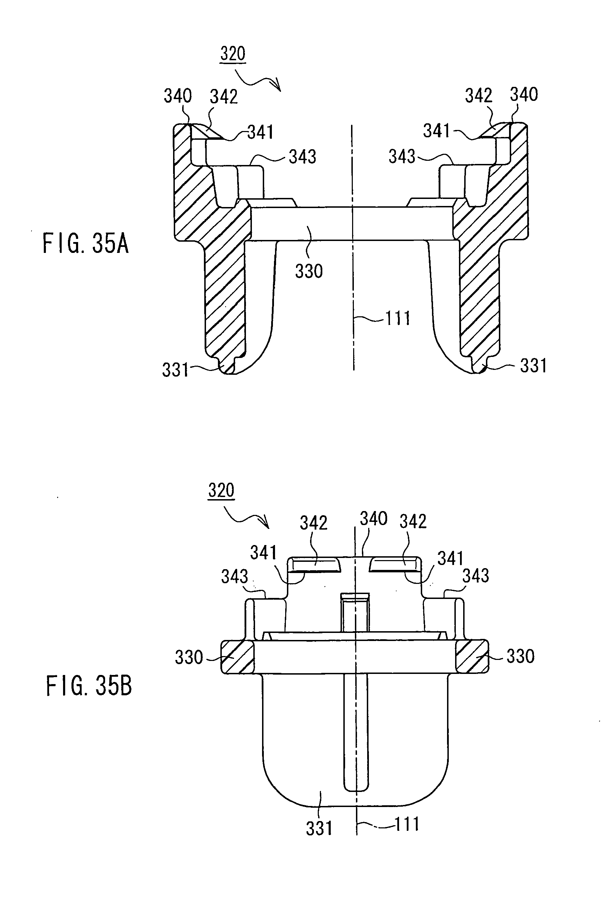

FIG. 33 is a perspective view of a handle 320 constituting the female connector according to Embodiment 3 of the present invention. FIG. 34A is a plan view of the handle 320, and FIG. 34B is a front view thereof FIG. 35Ais a cross-sectional view taken along a line 35A-35Ain FIG. 34A, and FIG. 35B is a cross-sectional view taken along a line 35B-35B in FIG. 34B. Since an adaptor constituting the female connector according to Embodiment 3 together with the handle 320 is the same as that described in Embodiment 1, the description thereof will be omitted. In FIGS. 35A and 35B, alternate long and short dashed lines 111 indicate the center axis of the adaptor described in Embodiment 1 to be inserted into the handle 320, and the center axis of the adaptor is matched with the center axis of the female connector of Embodiment 3 and the center axis of the handle 320. As described in Embodiment 1, the direction of the center axis 111 is defined as an up-and-down direction, and a side connected to the male connector will be referred to as an "upper side", and an opposite side thereof will be referred to as a "lower side".

The handle 320 includes a frame 330 in an elliptical shape, at the center of which a through-hole 322 is formed. The inner diameter of the through-hole 322 is larger than the outer diameter of the tube-shaped section 112 of the adaptor 110 and is smaller than the outer diameter of the flange section 113. Apair of flange holding sections 321 bulged upward are formed in the vicinity of the through-hole 322 on the upper surface of the frame 330. The pair of flange holding sections 321 are formed in an arc shape so as to surround the through-hole 322 on both sides in a major axis direction (a right-and-left direction on the drawing surface of FIG. 34A) of the frame 330 with respect to the through-hole 322. The diameter of the arc constituting the pair of flange holding sections 321 is smaller than the outer diameter of the flange section 113 of the adaptor 110.

Apair of clips 340 protruding upward and a pair of operation sections 331 protruding downward are formed at both ends in the major axis direction of the frame 330.

In each of the pair of clips 340, a pair of engagement tabs 341 protruding toward the clip 340 on the partner side and a pair of abutment surfaces 343 directed upward are formed on a surface (an inner peripheral surface) on the side opposed to the clip 340 on the partner side. The engagement tab 341 is formed at the upper end of the clip 340 or in the vicinity thereof. On the upper surface of the engagement tab 341, an inclined surface 342 inclined with respect to the center axis 111 is formed. The abutment surface 343 is positioned slightly below the engagement tab 341.

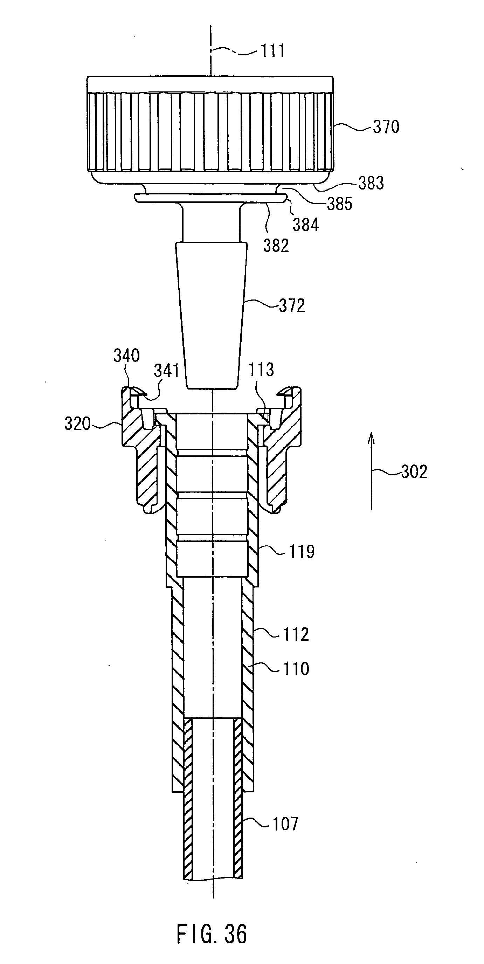

A method for connecting the female connector of Embodiment 3 to the male connector 370 will be described with reference to FIG. 36. In FIG. 36, a medical container including a port, to which the male connector 370 is connected, is not shown. The adaptor 110 is inserted into the through-hole 322 of the handle 320, and the lower surface 114 of the flange section 113 of the adaptor 110 is supported by the flange holding sections 321 of the handle 320. In this state, the outer peripheral surface of the frame 330 of the handle 320 is grasped with two fingers, and the adaptor 110 is installed on the tubular section 372 in the direction indicated by an arrow 302 so that the adaptor 110 covers the outer peripheral surface of the tubular section 372 of the male connector 370. In the same way as in Embodiment 1, the insertion section 119 of the adaptor 110 has flexibility and elasticity so that the insertion section 119 is deformed in accordance with the shape of the outer peripheral surface of the tubular section 372 of the male connector 370 and comes into tight contact with the outer peripheral surface of the tubular section 372 due to the elastic recovery force. On the other hand, the handle 320 has a high rigidity, so that the grasping force applied to the handle 320 in a diameter direction thereof by the two fingers will not increase the friction between the insertion section 119 of the adaptor 110 and the tubular section 372. Further, the flange section 113 is formed at a front end of the adaptor 110 in the direction indicated by the arrow 302, so that the adaptor 110 will not be buckled and deformed due to the force applied to the handle 320 in the direction indicated by the arrow 302 by the two fingers.

After the tubular section 372 of the male connector 370 is inserted deeply into the insertion section 119 of the adaptor 110, the handle 320 further is pushed in the direction indicated by the arrow 302 with respect to the male connector 370. The flange section 113 of the adaptor 110 is deformed elastically in the direction indicated by the arrow 302 by the flange holding sections 321 of the handle 320. Then, the inclined surfaces 342 formed at the tip end of each clip 340 of the handle 320 abut on the engagement protrusion 384 of the male connector 370, and the frame 330 is deformed elastically in a direction in which the upper ends of the pair of clips 340 separate from each other. Further, when the handle 320 is pushed in the direction indicated by the arrow 302 with respect to the male connector 370, the engagement tabs 341 of the clips 340 overpass the engagement protrusion 384 of the male connector 370. Then, the frame 330 is recovered elastically, and the engagement tabs 341 of the clips 340 are fitted in the undercut section 385 of the male connector 370. Simultaneously, the upper end faces of the clips 340 abut on the annular region 383 of the male connector 370, and the abutment surfaces 343 of the clips 340 abut on the lower surface of the platform 382 of the male connector 370. In this state, when the hand is taken off the handle 320, the handle 320 moves slightly in the direction opposite to the arrow 302 due to the elastic recovery force of the flange section 113 of the adaptor 111 that is deformed elastically in the direction indicated by the arrow 302, and the engagement tabs 341 of the clips 340 and the engagement protrusion 384 of the male connector 370 are engaged with each other. Thus, the handle 320 and the male connector 370 are engaged with each other, whereby the adaptor 110 can be prevented from coming off the tubular section 372. FIG. 37 shows a state in which the female connector and the male connector 370 are connected to each other. The engagement state between the engagement tabs 341 of the clips 340 and the engagement protrusion 384 of the male connector 370 cannot be cancelled unless the frame 330 is deformed elastically in a direction in which the upper ends of the pair of clips 340 separate from each other. Therefore, even when a small impact such as the touch of a hand is applied, the above engagement state will not be cancelled.

The female connector can be separated from the male connector 370 by performing the above operation in an opposite way. That is, the pair of operation sections 331 of the handle 320 are pinched with two fingers, and the frame 330 is deformed elastically in a direction in which the upper ends of the pair of clips 340 separate from each other. Consequently, the engagement between the engagement tabs 341 and the engagement protrusion 384 is cancelled, and the handle 320 moves in the direction opposite to the arrow 302 due to the elastic recovery force of the flange section 113 of the adaptor 110 that is deformed elastically in the direction indicated by the arrow 302. Then, the adaptor 110 is pulled out from the tubular section 372 of the male connector 370.

If the shape of the periphery of the engagement protrusion 384 including the platform 382, provided on the male connector 370, corresponds to the shape of the clips 340 provided on the handle 320, the handle 320 and the male connector 370 can be engaged with each other. That is, even if the shape, size, and the like of the tubular section 372 of the male connector 370 vary depending upon the manufacturers and specifications, the handle 320 and the male connector 370 can be engaged with each other.

On the other hand, in Embodiment 3, in the same way as in Embodiment 1, even if the shape, size (for example, a taper angle, an outer diameter, etc.) of the outer peripheral surface of the tubular section 372 of the male connector 370 vary depending upon the manufacturers and specifications, the insertion section 119 of the adaptor 110 is deformed elastically in accordance with the outer peripheral surface of the tubular section 372 of the male connector 370, so that the male connector 370 and the female connector can be connected to each other precisely without the leakage of the liquid-like substance.

Thus, in the case where the male connector has the engagement protrusion 384 corresponding to the shape of the clips 340 of the handle 320 in Embodiment 3, the adaptor 110 can be connected to the tubular section of the male connector, using the handle 320, and further, the handle 320 and the male connector can be engaged with each other.

On the other hand, in the case where the male connector does not have the engagement protrusion 384 corresponding to the shape of the clips 340 of the handle 320 in Embodiment 3, the handle 320 is used only for connecting the adaptor 110 to the tubular section of the male connector. In this case, it is preferred that the mechanism (see FIGS. 9A, 9B, 10A, and 10B) holding the handle 220, described in Embodiment 1, is provided on the adaptor 110 so that the handle 320 does not move to a position far away from the adaptor 110 after the adaptor 110 is connected to the tubular section of the male connector.

According to Embodiment 3, the handle 320 and the male connector 370 can be engaged with each other merely by moving the handle 320 in the direction indicated by the arrow 302 with respect to the male connector 370. Further, the engagement between the handle 320 and the male connector 370 can be cancelled merely by grasping the pair of operation sections 331 and moving the handle 320 in the direction indicated by the arrow 302.

Further, the lower surface of the platform 382 of the male connector 370 is matched with the lower surface of the engagement protrusion 384 (that is, the engagement protrusion 384 is provided along the lower surface of the platform 382), so that the height of the male connector 370 from the annular region 383 of the platform 382 can be reduced. Consequently, the height of the clips 340 from the abutment surfaces 343 also can be reduced. This can reduce the size in an up-and-down direction of the male connector 370 and the handle 320.

The above-mentioned embodiment is an example, and the present invention is not limited thereto.

Although the pair of engagement tabs 341 and the pair of abutment surfaces 343 are provided in one clip 340 of the handle 320, the number of the engagement tabs 341 and the abutment surfaces 342 to be provided in one clip 340 may be one or three or more, instead of being limited to two. Further, the abutment surfaces 343 can be omitted.

Although the flange holding sections 321 are formed as two separate parts in the vicinity of the through-hole 322 on the upper surface of the frame 330, the flange holding sections 321 may be continuous in an annular shape. Further, the flange holding sections 321 do not need to be bulged, and may be a flat surface common to the upper surface in the surrounding of the frame 330.

In the present embodiment, the description of Embodiment 1 can be adapted as it is or with obvious alterations added thereto appropriately.

### (Embodiment 4)

In Embodiment 4, a female connector in which an engagement shape for preventing an adaptor connected to a tubular section of a male connector from coming off the tubular section accidentally is provided on a handle, and a male connector provided with an engagement shape corresponding thereto will be described

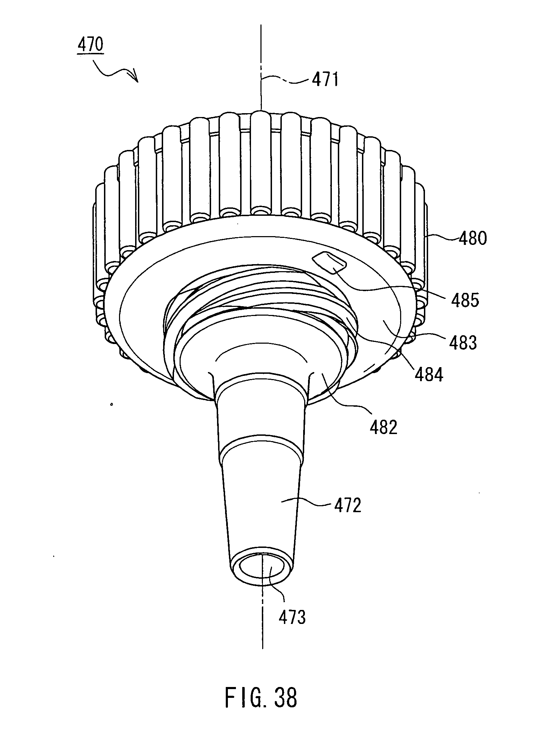

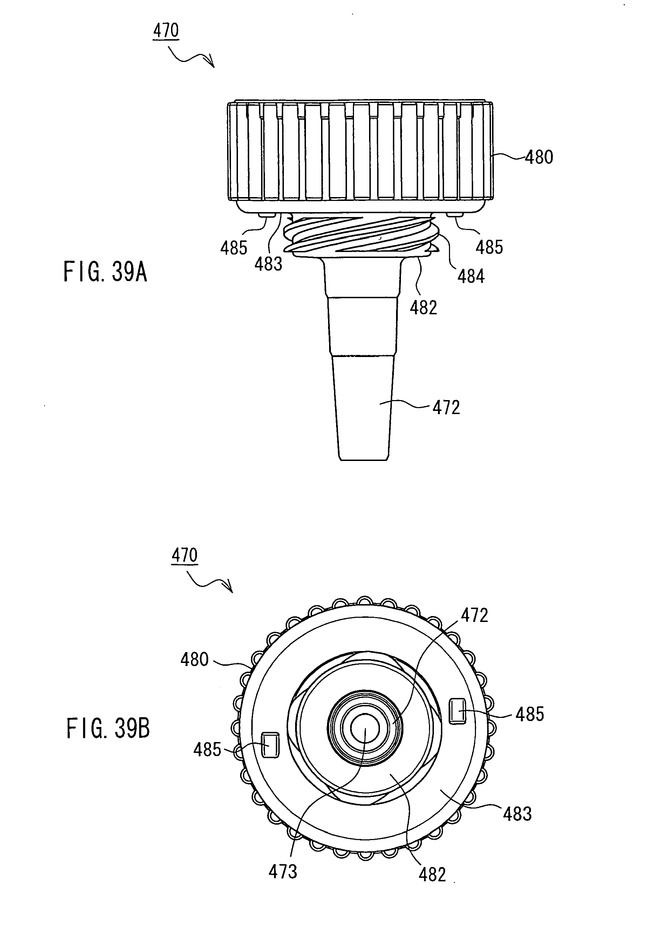

FIG. 38 is a perspective view of a male connector 470 according to Embodiment 4 of the present invention. FIG. 39Ais a front view of the male connector 470, and FIG. 39B is a bottom view thereof. In FIG. 38, alternate long and short dashed lines 471 indicate a center axis of the male connector 470. The direction of a center axis 471 is defined as an up-and-down direction, and an upper side on the drawing surface of FIG. 38 (a side connected to the port 930 of the medical container 910) will be referred to as an "upper side", and a lower side on the drawing surface of the figure (a side to which a female connector is connected) will be referred to as a "lower side".

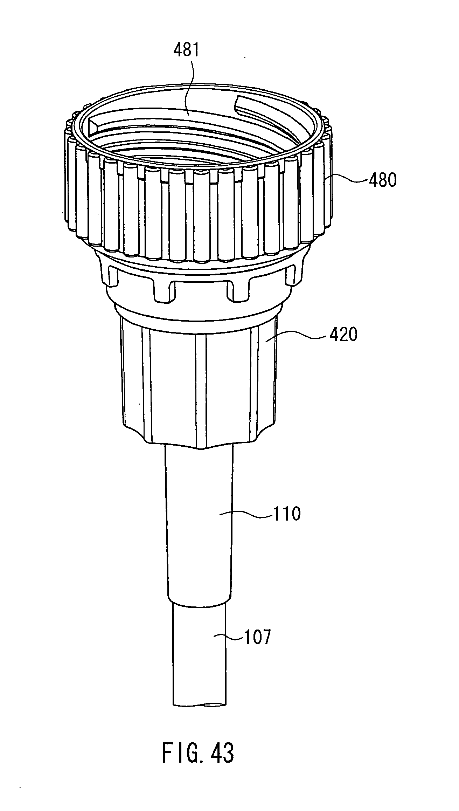

On the inner peripheral surface of a cap section 480, a female thread 481 to be screwed with the male thread section 936 (see FIG. 4) of the port 930 of the medical container 910 is formed (see FIG. 43). On the lower surface of the cap section 480, a circular region protrudes downward from the center compared with an annular region 483 in the surrounding of the circular region, whereby a platform 482 is provided. A tubular section 472 protruding downward is provided at the center of the platform 482. The outer peripheral surface of the tubular section 472 of the present example has three tapered surfaces (truncated cone surfaces), the outer diameters of which increase toward the cap 480, arranged in the direction of the center axis 471. In the tubular section 472, a through-hole 473, out of which the liquid-like substance in the pouch 920 flows, is formed.

On the outer peripheral surface of the platform 482, a male thread 484 is formed. The male thread 484 is a quadruple thread. In the quadruple thread, the distance (lead) by which the male thread 484 proceeds in the axis direction when making one rotation is four times an interval (pitch) in the axis direction of a screw thread. The annular region 483 is provided with a pair of protrusions 485 at symmetrical positions with-respect to the center axis 471.

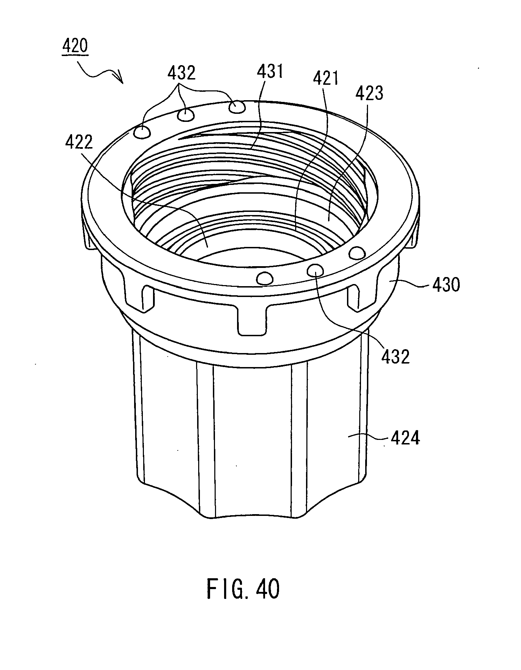

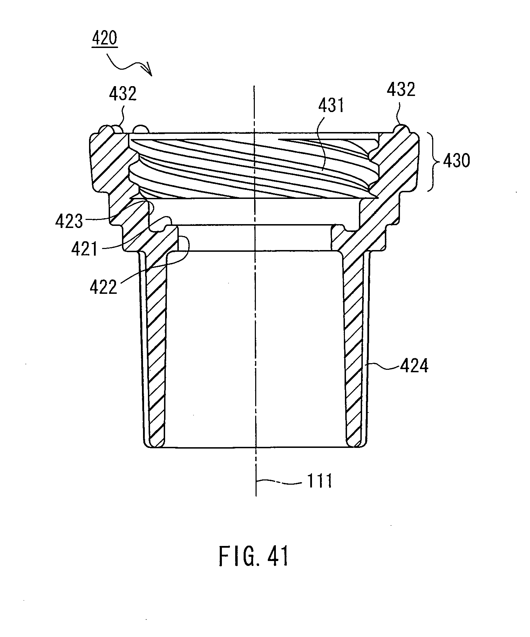

FIG. 40 is a perspective view of a handle 420 constituting the female connector according to Embodiment 4 of the present invention. FIG. 41 is a cross-sectional view taken along the center axis of the handle 420. Since an adaptor constituting the female connector according to Embodiment 4 together with the handle 420 is the same as that described in Embodiment 1, the description thereof will be omitted. In FIG. 41, alternate long and short dashed lines 111 indicate the center axis of the adaptor described in Embodiment 1 to be inserted into the handle 420, and the center axis of the adaptor is matched with the center axis of the female connector of Embodiment 4 and the center axis of the handle 420. As described in Embodiment 1, the direction of the center axis 111 is defined as an up-and-down direction, and a side connected to the male connector will be referred to as an "upper side", and an opposite side thereof will be referred to as a "lower side".

The handle 420 has a substantially cylindrical shape as a whole. The handle 420 has a large-diameter section 423 having an inner diameter larger than the outer diameter of the flange section 113 of the adaptor 110 in the vicinity of the upper end thereof, and a small-diameter section 422 having an inner diameter larger than the outer diameter of the tube-shaped section 112 of the adaptor 111 and smaller than the outer diameter of the flange section 113 on the lower side of the large-diameter section 423. Then, a flange holding section 421 is formed between the small-diameter section 422 and the large-diameter section 423. A grasping section 424 in a substantially cylindrical shape is provided on the lower side of the small-diameter section 422.

A screw-up section 430 in a substantially cylindrical shape is formed on the upper side of the large-diameter section 423. A female thread 431 is formed on the inner peripheral surface of the screw-up section 430. The female thread 431 is a quadruple thread capable of being screwed with a male thread 481 formed on the male connector 470. On the upper surface of the screw-up section 430, a plurality of protrusions 432 are formed.

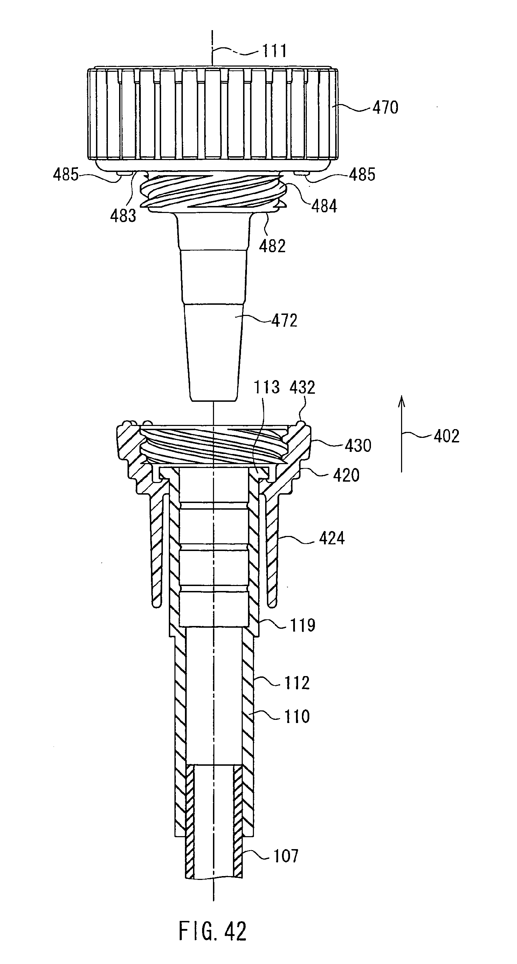

A method for connecting the female connector of Embodiment 4 to the male connector 470 will be described with reference to FIG. 42. In FIG. 42, a medical container including a port to which the male connector 470 is connected is not shown. The adaptor 110 is inserted into the handle 420, and the lower surface 114 of the flange section 113 of the adaptor 110 is supported by the flange holding section 421 of the handle 420. In this state, the outer peripheral surface of the grasping section 424 of the handle 420 is grasped with two fingers, and the adaptor 110 is installed on the tubular section 472 in the direction indicated by an arrow 402 so that the adaptor 110 covers the outer peripheral surface of the tubular section 472 of the male connector 470. In the same way as in Embodiment 1, the insertion section 119 of the adaptor 110 has flexibility and elasticity, so that the insertion section 119 is deformed in accordance with the shape of the outer peripheral surface of the tubular section 472 of the male connector 470 and comes into tight contact with the outer peripheral surface of the tubular section 472 due to the elastic recovery force. On the other hand, the handle 420 has a high rigidity, so that the grasping force applied to the handle 420 in a diameter direction thereof by the two fingers will not increase the friction between the insertion section 119 of the adaptor 110 and the tubular section 472. Further, the flange section 113 is formed at a front end of the adaptor 110 in the direction indicated by the arrow 402, so that the adaptor 110 will not be buckled and deformed due to the force applied to the handle 420 in the direction indicated by the arrow 402 by the two fingers.

After the tubular section 472 of the male connector 470 is inserted deeply into the insertion section 119 of the adaptor 110, the handle 420 further is pushed in the direction indicated by the arrow 402 with respect to the male connector 470, and the handle 420 is rotated clockwise around the center axis 111 with respect to the male connector 470. The male thread 484 of the male connector 470 and the female thread 431 of the handle 420 are engaged with each other, and the handle 420 rises. Finally, the upper surface of the screw-up section 430 of the handle 420 abuts on the annular region 483 of the male connector 470. At this time, the pair of protrusions 485 formed in the annular region 483 of the male connector 470 respectively are engaged with any of the plurality of protrusions 432 formed on the upper surface of the screw-up section 430 of the handle 420. Therefore, the screwed-together between the male thread 484 and the female thread 431 can be prevented from being loosened. Further, the flange section 113 of the adaptor 110 is deformed elastically in the direction indicated by the arrow 402 by the flange holding section 421 of the handle 420, and the elastic recovery force thereof generates friction between the male thread 484 and the female thread 431, so that the screwed-together state between the male thread 484 and the female thread 431 can be prevented further from being loosened. Thus, the handle 420 and the male connector 470 are engaged with each other, whereby the adaptor 110 can be prevented from coming off the tubular section 472. FIG. 43 shows a state in which the female connector and the male connector 470 are connected to each other. Since the male thread 484 and the female thread 431 are screwed with each other, and the above configuration is provided so as to prevent the screwed together state from being loosened, even when a small impact such as the touch of a hand is applied, the engagement state between the male thread 484 of the male connector 470, and the female thread 484 of the handle 420 will not be cancelled.

The female connector can be separated from the male connector 470 by performing the above operation in an opposite way. That is, the handle 420 is rotated counterclockwise around the center axis 111 with respect to the male connector 470. Consequently, the pair of protrusions 485 formed in the annular region 483 of the male connector 470 overpass the plurality of protrusions 432 formed on the upper surface of the screw-up section 430 of the handle 420, whereby the engagement state between the male connector 470 and the handle 420 is cancelled. By rotating the handle 420 further, the engagement between the male thread 484 of the male connector 470 and the female thread 431 of the handle 420 is cancelled. Then, the adaptor 110 is pulled out from the tubular section 472 of the male connector 470.

If the shape of the male thread 484 provided on the male connector 470 corresponds to the shape of the female thread 431 provided on the handle 420, the handle 420 and the male connector 470 can be engaged with each other. That is, even if the shape, size, and the like of the tubular section 472 of the male connector 470 vary depending upon the manufacturers and specifications, the handle 420 and the male connector 470 can be engaged with each other.

On the other hand, in Embodiment 4, in the same way as in Embodiment 1, even if the shape, size (for example, a taper angle, an outer diameter, etc.), and the like of the outer peripheral surface of the tubular section 472 of the male connector 470 vary depending upon the manufacturers and specifications, the insertion section 119 of the adaptor 110 is deformed elastically in accordance with the outer peripheral surface of the tubular section 472 of the male connector 470, so that the male connector 470 and the female connector 400 can be connected to each other precisely without the leakage of the liquid-like substance.

Thus, in the case where the male connector has the male thread 484 corresponding to the shape of the female thread 431 of the handle 420 in Embodiment 4, the adaptor 110 can be connected to the tubular section of the male connector, using the handle 420, and further, the handle 420 and the male connector can be engaged with each other.

On the other hand, in the case where the male connector does not have the male thread 484 corresponding to the shape of the female thread 431 of the handle 420 in Embodiment 4, the handle 420 is used only for connecting the adaptor 110 to the tubular section of the male connector. In this case, it is preferred that the mechanism (see FIGS. 9A, 9B, 10A, and 10B) holding the handle 220, described in Embodiment 1, is provided on the adaptor 110 so that the handle 420 does not move to a position far away from the adaptor 110 after the adaptor 110 is connected to the tubular section of the male connector.

According to Embodiment 4, the handle 420 and the male connector 470 can be engaged with each other, and the engagement thereof can be cancelled merely by rotating the handle 420 around the center axis 111 with respect to the male connector 470.

The above embodiment is an example, and the present invention is not limited thereto.

For example, the quadruple threads are used as the male thread 484 and the female thread 431; however, the present invention is not limited thereto. A multiple thread other than the quadruple thread may be used or a single thread may be used. It should be noted that, if the multiple thread is used, the male thread 484 and the female thread 431 can be engaged with each other with a small rotation angle of the handle 420.

The pair of protrusions 485 formed in the annular region 483 of the male connector 470 and the plurality of protrusions 432 formed on the upper surface of the screw-up section 430 of the handle 420, having a thread locking function, can be omitted.

In the present embodiment, the description of Embodiment 1 can be adapted as it is or with obvious alterations added thereto appropriately.

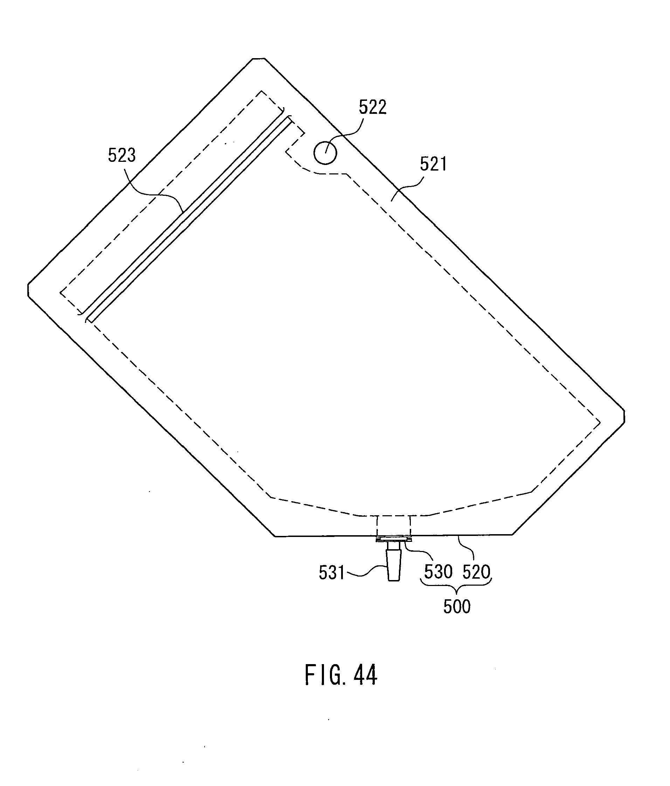

In Embodiments 1-4, as shown in FIG. 4, an example has been described in which the male connector 170 is provided integrally with the cap section 180 having the female thread section to be screwed with the male thread section 936 formed on the port 930 of the medical container 910; however, the present invention is not limited thereto. For example, as shown in FIG. 44, a tubular section 531 to be inserted into an adaptor of a female connector may be formed integrally with a port 530 to be joined to a pouch 520 by heat seal. That is, the port 530 can be considered as a male connector provided with the tubular section 531. In FIG. 44, reference numeral 500 denotes a medical container, 520 denotes a pouch, 521 denotes a seal region of two seats constituting the pouch, 522 denotes an opening used for hanging the medical container 500, and 523 denotes a fastener.

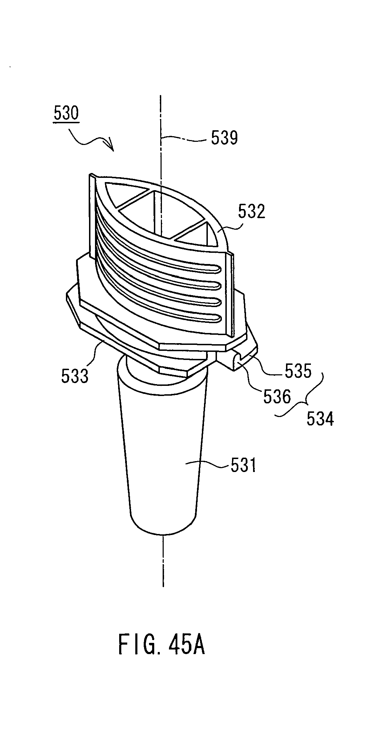

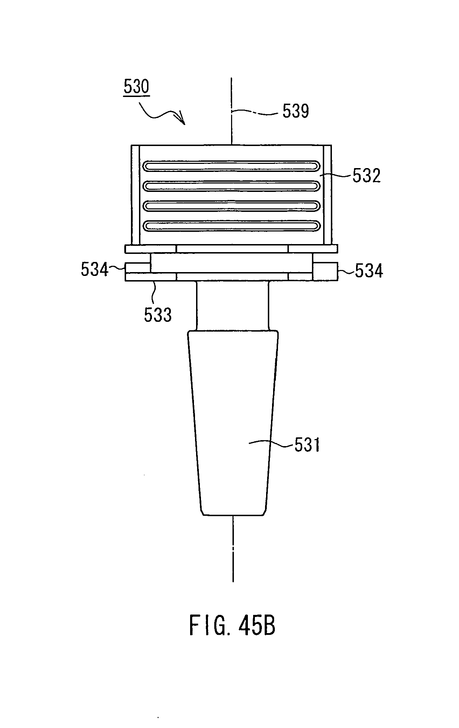

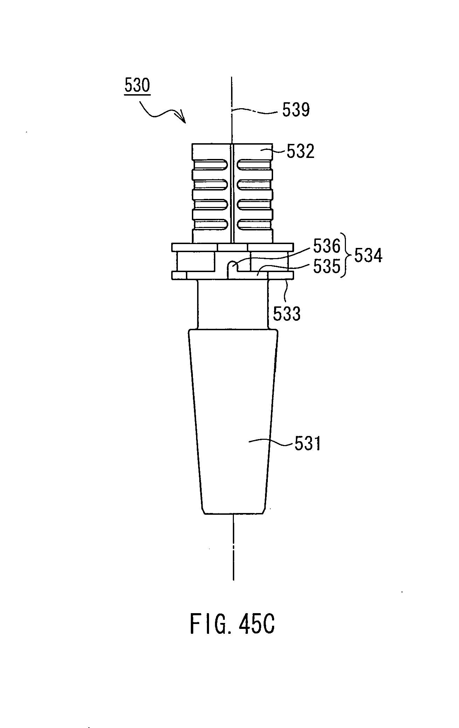

FIG. 45Ais a schematic perspective view of a male connector (port) 530 to be used for the medical container 500 in FIG. 44; FIG. 45B is a front view thereof and FIG. 45C is right side view thereof. In these figures, alternate long and short dashed lines 539 indicate a center axis of the male connector 530. The direction of the center axis 539 is defined as an up-and-down direction, and an upper side on the drawing surfaces of the figures (a side connected to the pouch 520) will be referred to as an "upper side" and a lower side on the drawing surfaces of the figures (a side to which the female connector is connected) will be referred to as a "lower side".

The male connector 530 includes a joint section 532 to be integrated with the pouch 520 by heat seal, while being sandwiched between edges of two sheets constituting the pouch 520, the tubular section 531 provided with a through-hole (not shown) out of which the liquid-like substance filling the pouch 520 flows, and an engagement plate (platform) 533 provided between the joint section 532 and the tubular section 531. The tubular section 531 protruding downward is provided at the center of the lower surface of the engagement plate 533. On the outer peripheral surface of the engagement plate 533, a pair of engagement tabs 534 are formed so as to protrude in a radial direction with respect to the center axis 539 at symmetrical positions with respect to the center axis 539. The engagement tab 534 includes an engagement chip 535 extending substantially in a horizontal direction and a lock protrusion 536 formed so as to protrude upward at one end of the engagement chip 535. The pair of engagement tabs 534 have a function similar to that of the pair of engagement tabs 284 formed on the male connector 270 described in Embodiment 2.

Although the male connector 530 has the pair of engagement tabs 534 that function similarly to the pair of engagement tabs 284 described in Embodiment 2, the male connector 530 may have a member that functions similarly to the engagement protrusion 384 described in Embodiment 3 or a member that functions similarly to the male thread 484 described in Embodiment 4.

In Embodiments 1-4, the case has been described where the male connector provided at a medical container and the female connector provided at one end of a transintestine nutrient set are connected to each other; however, the present invention is not limited thereto. For example, the present invention can be applied to the case where a male connector provided at one end of a tube through which a liquid-like substance passes and a female connector provided at one end of another tube are connected to each other.

The female connector described in Embodiments 1-4 can be provided at one end of a PEG tube. In this case, a male connector to be connected to the female connector can be provided at one end of a nutrient set to be connected to a container (for example, a pouch) storing a liquid-like substance such as a nutrient or a liquid diet. Since there are no specifications to be standards regarding the shape and size of the male connector to be connected to the PEG tube, it is highly significant that the present invention is applied to the PEG tube.

In order to keep the inside of the PEG tube clean, the PEG tube may be filled with diluted vinegar, when the liquid-like substance is not administered. In such a case, since an adaptor of the female connector is wet, even when the adaptor is connected to a tubular section of the male connector, the adaptor may come off the tubular section easily. Further, it is necessary to push out the liquid-like substance toward a patient in the case where the liquid-like substance has high viscosity. In this case, the adaptor of the female connector also is likely to come off the tubular section of the male connector due to the pressure applied to the liquid-like substance. Thus, in the case where the present invention is applied to the PEG tube, as described in Embodiments 2-4, it is preferred to provide an engagement shape to be engaged with the male connector on a handle of the female connector. Further, it is preferred that the male connector is provided with an engagement shape capable of being engaged with the engagement shape provided on the handle. This can prevent the adaptor of the female connector connected to the tubular section of the male connector from coming off the tubular section accidentally.

The embodiments described above are shown strictly for the purposes of clarifying the technical contents of the present invention; the present invention should not be interpreted only based on such specific examples and can be carried out in various modifications within the scope of the spirit of the invention and the claims, and should be interpreted in a broad meaning.

### Industrial Applicability

There is no particular limit to the field of the invention, and the present invention can be used, for example, in a female connector or a connector to be used for performing a transintestine nutrition therapy or an intravenous nutrition therapy. In addition, the present invention also can be used in a female connector, a connector, or the like to be used for dealing with a liquid-like substance such as food other than those for medical purposes.

## Claims

1. A female connector to be connected to a male connector having a tubular section, out of which a liquid-like substance flows, comprising:
an adaptor installed on an outer peripheral surface of the tubular section, and a handle fitted on the adaptor,
wherein the adaptor has a tube-shaped section having an opening at one end thereof, the tube-shaped section includes an insertion section into which the tubular section is inserted from the opening, and the insertion section has flexibility and elasticity,
the handle has a rigidity higher than that of the insertion section of the adaptor, and
there is provided movement limitation means limiting a range in which the handle is capable of moving relative to the adaptor from an end of the adaptor on an opposite side of the opening to an end thereof on the opening side in a center axis direction of the adaptor.

2. The female connector according to claim 1, wherein the movement limitation means includes a flange section provided at the end of the tube-shaped section of the adaptor on the opening side or in a vicinity thereof so as to protrude outward from the outer peripheral surface of the tube-shaped section, and a flange holding section provided on the handle so as to hold a surface of the flange section on an opposite side of the opening.

3. The female connector according to claim 1, wherein the movement limitation means further limits a range in which the handle is capable of moving relative to the adaptor from the end of the adaptor on the opening side to the end thereof on the opposite side of the opening in the center axis direction of the adaptor.

4. The female connector according to claim 3, wherein the movement limitation means includes an annular groove in a circumferential direction, provided on a surface of one of the tube-shaped section and the handle opposed to the other, and a fitting section provided on the other of the tube-shaped section and the handle so as to be fitted in the groove.

5. The female connector according to claim 4, wherein, when a width of the groove is defined as W 1 and a width of the fitting section is defined as W2 in the center axis direction of the adaptor, 1.0≤W1/W2≤ 1.5 is satisfied.

6. The female connector according to claim 3, wherein the movement limitation means includes a bonding section that bonds the adaptor to the handle.

7. The female connector according to claim 1, wherein an inner peripheral surface of the insertion section of the adaptor has a tapered surface whose inner diameter increases toward the opening.

8. The female connector according to claim 1, wherein an inner peripheral surface of the insertion section of the adaptor is provided with an annular protrusion in a circumferential direction.

9. The female connector according to claim 1, wherein an inner peripheral surface of the insertion section of the adaptor is provided with an annular difference in level in a circumferential direction, and the difference in level changes an inner diameter of the insertion section in a stepped shape so that the inner diameter of the insertion section on the opening side with respect to the difference in level becomes larger than the inner diameter of the insertion section on an opposite side of the opening with respect to the difference in level.

10. The female connector according to claim 2, wherein the adaptor is provided with a holding mechanism holding the handle.

11. The female connector according to claim 10, wherein the holding mechanism is a locking protrusion that protrudes outward from the outer peripheral surface of the tube-shaped section of the adaptor so as to abut on an end of the handle on an opposite side of the male connector.

12. The female connector according to claim 10, wherein the holding mechanism is a contact protrusion that protrudes outward from the outer peripheral surface of the tube-shaped section of the adaptor so as to abut on an inner peripheral surface of the handle.

13. The female connector according to claim 2, wherein a tube connected to an end of the adaptor on an opposite side of the opening is provided with a holding mechanism holding the handle.

14. The female connector according to claim 2, wherein the handle is provided with a holding mechanism so as to be held by a tube connected to an end of the adaptor on an opposite side of the opening or the adaptor.

15. The female connector according to claim 1, wherein the handle has an annular shape surrounding the adaptor without any rift.

16. The female connector according to claim 1, wherein an inner peripheral surface of the handle has a tapered surface whose inner diameter decreases toward the male connector.

17. The female connector according to claim 1, wherein an inner peripheral surface of the handle has a small-diameter section whose inner diameter is smaller than that of the other sections.

18. The female connector according to claim 1, wherein the handle is provided with a slit connecting an-upper end to a lower end thereof so as to be discontinuous in a circumferential direction of the adaptor.

19. The female connector according to claim 18, wherein a hinge is formed at a position to which the slit of the handle is opposed, and two halves constituting the handle are connected rotatably via the hinge.

20. The female connector according to claim 18 or 19, wherein the handle includes diameter expansion limitation means that limits a diameter expansion of the handle.

21. The female connector according claim 1, wherein the handle includes an engagement shape capable of being engaged with the male connector.

22. The female connector according to claim 21, wherein the handle includes a brim section that protrudes on the male connector side with respect to the movement limitation means, and the engagement shape is provided at the brim section.

23. The female connector according to claim 22, wherein an inner peripheral surface of the brim section opposed to a center axis of the handle includes an engagement wall that extends in a circumferential direction, a concave portion provided on an opposite side of the engagement wall with respect to the male connector, and a convex portion provided adjacent to the concave portion on either one side in the circumferential direction with respect to the concave portion, and the engagement shape includes the engagement wall.

24. The female connector according to claim 1, wherein the handle includes a frame having the movement limitation means, a pair of clips protruding on the male connector side with respect to the frame, and a pair of operation sections protruding on an opposite side of the male connector with respect to the frame, wherein a surface on a side opposed to each of the pair of clips is provided with an engagement tab, and when the frame is deformed elastically so as to narrow an interval between tip ends of the pair of operation sections, an interval between tip ends of the pair of clips is widened.

25. The female connector according to claim 1, wherein the handle includes a female thread on the male connector side with respect to the movement limitation means.

26. The female connector according to claim 1, wherein the liquid-like substance is a transintestine nutrient in a transintestine nutrition therapy

27. A connector comprising a male connector having a tubular section, out of which a liquid-like substance flows, and a female connector to be connected to the male connector,
wherein the female connector is the female connector according to any one of claims 1 to 26.

28. The connector according to claim 27, wherein, when the tubular section of the male connector is inserted into the insertion section of the adaptor of the female connector, the insertion section is squeezed by the tubular section and the handle.

29. The connector according to claim 27, wherein the male connector and the handle of the female connector are provided with engagement shapes that are engaged with each other.

30. A connector comprising a male connector having a tubular section, out of which a liquid-like substance flows, and a female connector to be connected to the male connector,
wherein the female connector is the female connector according to claim 23,
the male connector has a platform in which the tubular section protrudes from a center thereof,
an outer peripheral surface of the platform is provided with an engagement tab protruding in a radial direction with respect to a center axis, and
the engagement tab is stored in the concave portion of the handle, and the engagement tab is engaged with the engagement wall of the handle.

31. The connector according to claim 30, wherein the engagement tab of the male connector abuts on the convex portion of the handle to regulate a rotation of the handle with respect to the male connector.

32. The connector according to claim 30, wherein the engagement tab is provided along a surface of the platform, on which the tubular section is provided.

33. A connector comprising a male connector having a tubular section, out of which a liquid-like substance flows, and a female connector to be connected to the male connector,
wherein the female connector is the female connector according to claim 24,
the male connector has a platform in which the tubular section protrudes from a center thereof
an engagement protrusion protruding in a radial direction with respect to a center axis is provided continuously over the outer peripheral surface of the platform, and
the engagement tab of the handle is engaged with the engagement protrusion.

34. The connector according to claim 33, wherein the engagement protrusion is provided along a surface of the platform, on which the tubular section is provided.

35. A connector comprising a male connector having a tubular section, out of which a liquid-like substance flows, and a female connector to be connected to the male connector,
wherein the female connector is the female connector according to claim 25,
the male connector has a platform in which the tubular section protrudes from a center thereof,
a male thread is formed on an outer peripheral surface of the platform, and
the female thread of the handle is screwed with the male thread.

36. The connector according to any one of claims 27 to 35, wherein the connector is provided on a line connecting a container storing a transintestine nutrient in a transintestine nutrition therapy to a patient.
